Europäisches Patentamt

(19) European Patent Office

Office européen des brevets

(11) Publication number: **0 062 495**
**B1**

(12) **EUROPEAN PATENT SPECIFICATION**

(45) Date of publication of patent specification: **11.11.87**

(21) Application number: **82301701.7**

(22) Date of filing: **31.03.82**

(51) Int. Cl.⁴: **A 61 F 5/44**

(54) Absorbent pads, incontinence care products and methods of production.

(30) Priority: **31.03.81 US 249318**
**31.03.81 US 249319**
**15.04.81 US 254314**
**28.05.81 US 267833**
**10.03.82 PCt/us82/00312**

(43) Date of publication of application:
**13.10.82 Bulletin 82/41**

(45) Publication of the grant of the patent:
**11.11.87 Bulletin 87/46**

(84) Designated Contracting States:
**AT BE CH DE FR GB IT LI LU NL SE**

(56) References cited:
**AT-B- 317 413**
**DE-A-2 554 640**
**DE-B-2 559 606**
**US-A-3 878 283**

(73) Proprietor: **Mitchell, James G.**
**c/o Principle Business Enterprises, Inc. Pine Lake**
**Industrial Park**
**Dunbrige Ohio 43414 (US)**
(73) Proprietor: **Mitchell, Winalee G.**
**c/o Principle Business Enterprises, Inc. Pine Lake**
**Industrial Park**
**Dunbrige Ohio 43414 (US)**

(72) Inventor: **Mitchell, James G.**
**110 Secor Woods Lane**
**Perrysburg Ohio, 43551 (US)**
Inventor: **Mitchell, Winalee G.**
**110 Secor Woods Lane**
**Perrysburg Ohio 43551 (US)**
Inventor: **Pfaff, Alan R., Jr.**
**3512 MacNichol Trail**
**Orchard Lake Michican, 48033 (US)**
Inventor: **Bell, Jerry L.**
**3840 Hidden Creek Road**
**Rochester Michigan 48063 (US)**
Inventor: **Strauss, Robert E.**
**510 East Boundary Street**
**Perrysburg Ohio, 43551 (US)**
Inventor: **Keinath, David P.**
**676 Fairledge**
**Lake Orion Michigan 48085 (US)**

Courier Press, Leamington Spa, England.

EP 0 062 495 B1

**0 062 495**

Inventor: **Strader, Charles J.**
**26851 West River Road**
**Perrysburg Ohio 43551 (US)**
Inventor: **Bollinger, William C.**
**3900 Old Creek Road**
**Troy Michigan 48084 (US)**

(74) Representative: **Bowman, Paul Alan et al**
**LLOYD WISE, TREGEAR & CO. Norman House**
**105-109 Strand**
**London WC2R OAE (GB)**

## Description

Technical Field

This invention relates to absorbent pads useful for the care of urinary incontinence and other applications, combinations of such pads with briefs, and methods of making such pads.

Background of the Invention

The urinary incontinence care products available on the market today range from enlarged versions of the familiar disposable infant diapers to cumbersome pants arrangements, with many variations between these extremes. Some of the products are effective against leakage and odor emission, but are difficult to dispose of and/or cumbersome and/or heavy and/or uncomfortable to wear for long periods whether wet or dry. Others, which are less cumbersome, heavy or uncomfortable, appear to be less effective against leakage and odor emission. Thus a need exists for urinary incontinence care products or systems which are smooth and close fitting rather than bulky, are readily disposable, exert relatively little localized pressure on the skin and yet effectively stay in place, and are light in weight and effective against leakage and emission of odors.

The present invention arose in part from attempts to produce satisfactory urinary incontinence care pads from absorbent materials which are capable of being formed into layers and which are highly absorbent, i.e. capable of absorbing at least about 5, more particularly at least about 15, and most preferably at least about 35 times their own weight of water or urine. Such layers are known from DE—A—2559606 and AT—B—317413. One example of such layers of highly absorbent material is a commercially available "sandwich" comprising a central film of polymeric material, such as for instance lightly cross-linked, partially saponified polyacrylate or polyacrylamide that is water absorbent (including water-swellable or -soluble), having a cellulosic fiber layer on each of its major surfaces.

Heretofore, in the attempt to produce absorptive pads for the care of patients subject to urinary incontinence, it has been assumed that at least some patients would require a pad having both a large ultimate absorption capacity and a relatively high rate of absorption. For example, in severe applications, where sudden and major evacuations of liquid are likely, it is believed that a urinary incontinence care pad should be capable of absorbing, without substantial run off or loss of liquid, at least about 150, or 200, or 250 ccs. or more of urine at evacuation rates of at least 5 or 10 or more ccs. per second, and that the pad should have the foregoing absorption rate coupled with an ultimate capacity of at least about 200, or 250, or 300 or more ccs.

Heretofore, attempts to obtain the desired capacity and rate by forming pads from commonly available high-absorbency sheet materials have proved disappointing. In those instances where high capacities were obtained, absorption rates proved inadequate. Thus, it has been indicated that when the above-mentioned high absorbency sheet materials are present in the pad in sufficient amounts to constitute about 50% or more of the total absorption capacity of the pad, inadequate absorption rates result.

Heretofore, various attempts have been made to improve the absorption properties of the above-mentioned highly absorptive materials. It is reported that the manufacturer of one form of such material considered the forming of holes in the material. However, it is understood that the attempt was abandoned due to fear of loss of absorbent material and as consequence of punching pieces of absorbent material out of the sheet, with consequent loss of ultimate absorption capacity. It is also understood that there was some experimentation with forming small pinpricks or slits in such layers of highly absorptive materials, but these proved to be of little if any benefit.

From the foregoing, it may be seen that a need remains for rate-enhanced, liquid absorbent pads having high absorption capacity, and if such pads are to be used in the care of urinary incontinence, there is a need for them to be compact and readily disposable, such as by flushing in a common toilet. The principal objectives of the present invention are to fulfill the above needs.

Absorption Rate Test

A simple test was devised to determine the absorption rates of composites comprising high absorbency layers, e.g. sheets, such as for example the above-described "sandwich" composites comprising partially saponified lightly cross-linked polyacrylate or polyacrylamide films. According to this test procedure, test liquid is poured at a controlled rate and in a predetermined amount over successive samples of pads supported on a mild incline, (e.g. about 15° to 20° from horizontal). The rate and amount are selected to substantially exceed the absorption rate capabilities of the samples, and the amount of test liquid which runs off each pad before it can be absorbed is recorded and subtracted from the total amount poured. The difference in each case indicates the absorption rate capabilities of the sample pad. Exploratory research performed with the aid of the foregoing procedure surprisingly resulted in the discovery of how to produce both excellent rate characteristics and more than adequate ultimate capacity in absorbent pads, resulting in improved pads which are useful, among other things, for the care of urinary incontinence.

According to the invention, there is provided an absorbent pad including at least one absorptive layer containing water-absorbent material including water-swellable material, having the capacity to absorb at least five times its own dry weight of clean water or other aqueous liquid in which at least one absorptive layer provides at least 50% of the total liquid absorption capacity of the pad, and has openings which include hinge and flap members at the perimeters of the openings, the flap members extending outwardly from said at least one absorptive layer. At least one such absorptive layer in the pad comprises water-swellable material, which may be polymeric or non-polymeric, said material having the capability of absorbing at least about 5, preferably at least about 15 and more preferably at least about 35 times its own dry weight of clear water or other aqueous liquid.

The water-absorbent material is present in the pad in a sufficient amount so that at least about 50% of the total liquid absorption capacity of said pad is present in said water-absorbent material. The pad may also include other layers of non-absorbent material or of material having a lower absorption capacity per unit weight than the first-mentioned absorbent material.

The amount of the first-mentioned absorbent material is preferably sufficient so that it constitutes at least about 60%, and more preferably from about 65 to 80%, of the total liquid absorption capacity of the pad. Said forementioned absorbent material may be present in an amount sufficient to provide even 90% or higher of the total liquid absorptive capacity of the pad, especially where lower absorption rates than may be desired for urinary incontinence devices can be tolerated.

According to a particularly preferred embodiment of the present invention, the pad is formed from one or more absorptive layers which include undulations for increasing the quantity of absorptive layer material which is available for absorption beneath a given area of the liquid absorption surface of the pad, or for assisting in retaining restricting or directing the flow of liquid. Any suitable arrangement may be employed, a number of merely illustrative examples being provided by British published patent application 2,017,509 and by U.S. Patents 3,525,337, 3,610,244, 3,653,382, 3,865,112, 3,903,889, 3,968,798 and 4,176,667.

According to one of the aforementioned optional pad embodiments an absorbent sheet material may be formed into an elongated shaped pad of greater width than thickness, having an upper major surface and a lower major surface, at least one of which is adapted for placement adjacent a wearer's body for receipt of urine. The absorbent sheet material is present in the pad in one or more layers which, as viewed in cross section, having an undulating characteristic. The undulations formed in the sheet material may be of any shape or orientation which is suitable to enhance the total effective area of absorbent sheet material available for absorption beneath a given area of the upper or lower major surfaces.

The above-described pad with undulating sheet material may include a wide variety of undulations running in one or more varying directions. Preferably the undulations are arranged in the pad, and the pad is so positioned in briefs or other suitable holding garments or devices, so that the furrows and peaks or ridges formed by the undulations extend between the wearer's legs in a direction from front to rear. Also, the undulations may be produced with or without creasing of the absorbent material. Moreover, the undulations may be produced with varying forms, such as by crinkling, pleating, or forming serpentine loops. According to one embodiment, the absorbent sheet material is pleated with folds which have inner creases and outer edges. These folds alternate in opposite directions to form a plurality of panels of substantially varying or preferably uniform width which are interspersed with said folds. The folds may be and preferably are arranged across substantially the entire width of the pad, so that the edges of alternate folds are presented upwardly at or adjacent one of the above-mentioned major surfaces and the edges of intervening folds are presented downwardly at or adjacent the other of said major surfaces.

At least one layer comprising said absorbent material is provided with openings for appreciably enhancing the rate of liquid absorption of the pad. More specifically, the openings preferably have a sufficient average open area per opening and are distributed throughout a substantial portion of the available surface of the absorptive layer to provide an overall ratio of the total open area of said openings to the area of said portion (without deduction for the open area) in the range of about 0.002 to about 0.25, more particularly about 0.002 to about 0.20, preferably about 0.004 to about 0.15 and still more preferably about 0.008 to about 0.08. The open area per opening may for example be in the range of about 0.02 to about 1.3, more preferably about 0.05 to about 1 and more preferably about 0.15 to about 0.8 cm². The number of openings may for example be in the range of about 0.006 to about 0.3 or 0.4, more preferably about 0.006 to about 0.25, still more preferably about 0.01 to about 0.15 and most preferably about 0.1 to about 0.15 openings per cm² of the area of that portion of the material in which the openings are provided, it being understood that the openings may be present in all or only a portion of the absorptive layer comprising absorbent material and that the openings may be distributed throughout said portion or layer in a random or substantially uniform manner, or in a non-uniform manner intended to maximize rate of absorption in a particular section of the layer where extra absorption is desired or required. Where the openings are arranged in one or more discrete arrays which are collectively distributed over only a portion of the entire area of the layer, the area of said portion may be defined as the total area delineated by first tracing line segments intersecting with the centers of the outermost openings in each array to define an individual "field" for each array, and then adding to each field a border equal in width to the average spacing of all openings in its respective array of openings. In those instances in which the openings are provided throughout the available area of an absorptive layer, the total surface area of the layer (without deduction for the open area) may be used. The openings referred to above may generally be of any shape provided that they have significant open area and are therefore to be distinguished from slits, pinpricks or other openings which have been formed without either removal or at least folding back of material to form a significant open area. In accordance with the invention, the openings are formed by folding back a cut portion to form a hinge and flap construction which retains the absorption capacity of the "hole" material in the resultant pad.

According to a preferred embodiment, the absorptive layer and other components of the pad are selected from among materials which do not retain their structural integrity when mildly agitated in liquid, such as for example the mild agitation normally encountered within the bowl of a common reservoir-type household toilet wherein the hydrostatic head of the water in the reservoir relative to that in the bowl under static conditions, is about 60 cm. Thus, the pad preferably will have sufficient capability of undergoing appreciable or substantial disintegration under such agitation so that it is "flushable" i.e. it can readily be disposed of in such toilets with little or no danger of clogging.

According to a particularly preferred optional embodiment of the invention, any of the above-described pads or pads of other designs are combined with an elongated sheath portion, the latter being of suitable length and width and preferably having at least one opening or openable portion to facilitate removing wet pads therefrom. The sheath may or may not be, but preferably is, free of permanent connections between itself and the pad, but is of such width as to closely surround the pad. According to a particularly preferred embodiment, the sheath is of greater length than the pad and thus includes one or more extensions which extend beyond one or both ends of the pad for holding the pad in place in a brief or other holder.

The sheath portion may also be flushable but, according to one preferred embodiment of the invention, the sheath portion constitutes a water-pervious material which substantially retains its structural integrity on exposure to human urine. Particularly preferred for the sheath portion are non-woven or woven fabrics having sufficient open area between their fibers, strands or filaments and being formed of sufficiently non-absorbent material such as to prevent absorption of any substantial quantity of liquid by the sheath, e.g. polyester and/or polyolefin fibers, strands or filaments. A sheath portion so formed constitutes a convenient means for transporting a used absorbent pad from a pair of briefs or other holder to a disposal container or toilet. Such sheaths could in some cases be launderable and reuseable with disposable pads.

The above-described pads may be employed with or without briefs and have application to uses other than care of urinary incontinence.

In the care of urinary incontinence one may employ one or more absorbent pads capable of absorbing urine in the desired amounts and at the desired rate. For example one may employ a plurality of pads having in combination the desired capacity and absorption rate. It is however preferred to employ a single pad possessing the desired capacity and absorption rate. For example, in light duty applications, it is recommended that the pad or pads have an overall, ultimate absorption capacity of at least about 125, more preferably at least about 150 and still more preferably at least about 175 ccs (cubic centimeters) of natural or synthetic urine. In more severe applications, for example where sudden and major evacuations are likely, it is recommended that the pad be capable of absorbing, without substantial run-off or loss of liquid, at least about 150, more preferably at least about 200, and still more preferably at least about 250 ccs of natural or synthetic urine at an evacuation or application rate of at least 5 and preferably at least 10 ccs per second. It is further recommended that pads for such severe applications should have an overall or ultimate absorption capacity of at least about 200, more preferably at least about 250, and still more preferably at least about 300 ccs. In either case, ultimate capacity should be measured by applying test liquid at or below the maximum rate at which the pad or pads will accept liquid.

The urinary incontinence care brief employed in the present invention comprises a belt portion and a holder portion. The belt portion has posterior, side and front sections and is of sufficient length for extending about the posterior, sides and front of a wearer's abdomen. The holder portion has rearward, central and forward zones. These zones are of sufficient combined length for extending from the posterior section of the belt portion between the legs of the wearer to the front section of the belt portion. The brief also includes retaining means for removably retaining an absorbent pad in position upon said holder portion for absorbing urine evacuated by the wearer.

The method of the present invention comprises forming an absorbent pad comprising at least one but preferably a plurality of layers of material including at least one absorptive layer, of water absorbent material having the capability of absorbing at least five times its own dry weight of water or other aqueous liquid and having openings therein in which the water absorbent material represents at least 50% of the total liquid absorption capacity of the raw materials processed into the pad and the method comprises forming said openings by cutting around a portion but not the entire periphery of a closed curve for forming hinge and flap members, displacing said hinge and flap members outwardly to form a significant open area, and cutting the resulting pad stock transversely into sections to form pads.

The absorptive layer in the pad comprises water-swellable material, which may be polymeric of non-polymeric, said material having the capability of absorbing at least about 5, preferably 15 and more preferably at least about 35 its own dry weight of clear water or other aqueous liquid. At least one layer comprising said absorbent material is provided with openings, as above described, for appreciably enhancing the rate of liquid absorption of the pad.

Such openings are formed in a randomly distributed or patterned manner in the absorptive layer. A plurality of bends is formed in said layer running lengthwise thereof and spaced at intervals across the width of said layer, for forming said layer into an elongated pad stock of greater thickness and narrower width than said layer. The pad stock is eventually cut transversely at longitudinal intervals to form pads of predetermined length therefrom.

According to the invention, the above-mentioned openings are formed by cutting through material of said layer or layers along a portion but not all of the perimeter of the respective openings, thereby forming of said layer(s) one or more hinge and flap members at the respective openings. By means of force applied through the resultant openings, said flap members are displaced outwardly relative to the layer(s). Preferably, said flap members are then also displaced further in a direction inward relative to said layer(s) of absorbent material and the flap members are pressed against portions of one or more layers adjacent the respective hinges while leaving said openings substantially open. The first-mentioned displacement is preferably performed by means of members projected through the resultant openings, displacing the flap members outwardly relative to said layer(s). A preferred mode of performing the second displacement is rolling said layer(s) between rollers, thereby causing further displacement of the flap members in a direction inward relative to said layer(s).

A particularly preferred mode of operation is longitudinally advancing the absorbent layer of material in strip form to contact a first cutting roller which cuts the openings in the hinge and flap configuration and continuing the advance of the cut strip across the surface of a second roller with outwardly projecting members driven in synchronization with the cutting roller, to perform the first-mentioned displacement. The strip is then preferably rolled between rollers as described above.

By forming the hinge portions by cutting lines which, as viewed with the layer(s) in plan view, gently and smoothly curve past the edges of the hinge portions to ends which diverge from one another, one can inhibit tear propagation in the layer(s) in and/or adjacent the hinge members. In this connection it is considered beneficial if the gently, smoothly curved portions of the cutting lines include the transitions from the openings per se to positions alongside the hinge portions and/or if the ends of the cutting lines diverge from one another in opposite directions, especially parallel to the bending lines of the hinges. Where coincident hinge and flap type openings are formed simultaneously in the several layers of a moving composite and the several flaps at each opening through the composite are displaced outwardly together, they cooperate to bind the layers together and promote coordinated movement of the several layers. This is particularly useful where some of the layers are caused to move by directly applied driving force and others, for example inner layers in the composite, receive such driving force indirectly, i.e. through frictional engagement with other layers. For best results in attaining such binding, it is recommended that the hinge be formed with a lesser width, measured along the hinge bending line, than the maximum width of the opening, measured parallel to said bending line. When the hinge is of lesser width than the opening and the flap member is displaced to project away from the composite, it can then effectively lock together the layers in the composite during transport. When the hinge members are formed by cutting lines which curve in the above-described manner, this imparts extra strength to the hinge members which can be useful in performing the above-described binding function.

Another preferred embodiment of the invention includes forming a plurality of bends in the above-mentioned layer(s) running lengthwise thereof and spaced at intervals across the width of said layer(s), for forming said layer(s) into an elongated pad stock of greater thickness and narrower width than said layer(s). Best results have been obtained by bending at least one layer as it advances longitudinally in strip form through two arrays of rails or other forming members which, as scanned from edge to edge in transverse cross-section, cause the strip to contract laterally while alternatively displacing adjacent portions of the contracting strip in opposite directions perpendicular to the major surfaces of the strip, thereby forming an undulating or zig-zag pattern. When the previously described first displacement (and second displacement, if any) are performed in such a manner that the flaps are left extending away from the layer(s) from which they are formed, and the layer or layers are formed into an undulating pattern as above described, the flaps can act as spacers between adjacent undulations for promoting rapid access of incoming liquid to the length and depth of the undulations, thus promoting a high absorption rate.

According to another preferred embodiment, the method includes bringing pad stock prior to or after cutting of the pad stock, into contact with sheath material, described above, which is wider than said pad stock, causing the pad stock and sheath material to advance together longitudinally and bending the sheath material along a longitudinal line or lines for surrounding the pad stock as viewed in transverse cross-section.

When it is desired to form a sheath with extensions for securing and handling the completed pad, as mentioned above, the above-described transverse cutting of the pad stock is performed prior to contacting the pad stock and sheath material. Then the cut sections of pad stock are advanced into contact with the sheath material while the latter is advancing longitudinally at an accelerated rate relative to the rate of advancement of the pad stock during cutting, thereby causing the sections of pad stock to travel longitudinally in contact with the sheath material with a predetermined space between these sections which is about twice the length of the desired sheath extensions.

When the pad stock is cut transversely into sections as described above, a preferred embodiment of the invention includes applying compression during cutting to a narrow band of material adjacent the trailing edge of each cut. Sufficient compression is applied for causing collapsed and overlapping undulations and/or other layers in the pad stock to cling to one another with sufficient adherence to form a coherent leading edge at the front of each cut pad section which retains substantial structural integrity during subsequent advancement of the pad sections. Then the pad sections with coherent leading edges are advanced longitudinally into contact with sheath material while the latter is advancing longitudinally at an accelerated rate relative to the rate of advancement of pad stock during the transverse cutting of the pad stock. Thereafter, the pad stock sections travel longitudinally with the sheath material and the sheath material is cut transversely in the above-described manner.

The method invention includes a preferred mode of completing a sleeve-like sheath by the operation of securing its marginal edges together in overlapping or other relationship by any suitable technique such as sewing, or bonding through fusion, and/or adhesively bonding adjoining portions of such edges. According to a preferred embodiment the sheath, with continuous running length pad stock or with cut pad stock sections within it, is advanced longitudinally in strip form and the advancing marginal edges of the sheath material are brought into contact with one another and are caused to project outwardly relative to the nearest major surface of the pad stock during, and possibly also prior to, such securing operation.

Another optional but preferred embodiment commences subsequent to formation of the pad stock in strip form but prior to application of the sheath material. The pad stock is brought into contact with liquid permeable material in strip form which is wider than the pad stock. The pad stock and liquid permeable material are then advanced together longitudinally and the permeable material is bent along one or more longitudinal lines for encircling the pad stock as viewed in transverse cross-section, thereby forming a wrap of such permeable material around the pad stock. The above-described cutting operation is then applied to said pad stock and wrap together to form pads of predetermined length. The resultant wrapped pads are brought into contact with a strip of sheath material which is wider than said pads, the sheath material being bent along a longitudinal line or lines for encircling the pads as viewed in transverse cross-section. The marginal edges of the sheath material are then secured to one another for forming sleeve stock surrounding the wrapped pads. Optionally, transverse seals are formed in said sleeve stock adjacent at least one end of each pad.

Very rapid production of pads may be attained by an optional but preferred embodiment which includes advancing said layer of absorptive material longitudinally in strip form while making the randomly distributed or patterned openings therein at locations distributed substantially throughout the length of the strip; forming said openings by cutting through said strip along a portion but not all of the perimeter of the respective openings, thereby forming of said strip material one or more hinge and flap members at the respective openings; by means of force applied through the resultant openings, displacing said flap members outwardly relative to said strip; causing further displacement of said flap members in a direction inward relative to said strip and pressing said flap members against portions of the strip adjacent the respective hinges while leaving said openings substantially open; while advancing said strip longitudinally, forming a plurality of bends in said strip running lengthwise thereof and spaced at intervals across the width of said strip, for forming said strip into an elongated pad stock of greater thickness and narrower width than said strip; subsequent to formation of said pad stock, while advancing said pad stock longitudinally, bringing into contact with said pad stock a strip of liquid permeable material which is wider than said pad stock, causing said pad stock and liquid permeable material to advance together longitudinally, and bending the liquid permeable material along longitudinal lines for encircling the pad stock as viewed in transverse cross-section, thereby forming a wrap of liquid permeable material around said pad stock; and, while advancing said pad stock and wrap together longitudinally, cutting pads of predetermined length therefrom.

It is also optional but preferred to combine the operations of the preceding paragraph with further steps for rapidly forming sheaths with extensions about the resultant pads. Thus, subsequent to the cutting of said pads, they are brought into contact with a strip of sheath material which substantially retains its structural integrity when exposed to liquid and which is wider than said pads. The pads and sheath material are caused to advance together longitudinally at an accelerated rate relative to the rate of advancement of said pad stock and wrap during cutting, thereby causing said pads to travel longitudinally with a predetermined space therebetween which is about twice the combined length of sheath extensions to be formed subsequently. This embodiment also includes: bending the sheath material along a longitudinal line or lines for encircling the pads as viewed in transverse cross-section; continuously securing the sheath material in surrounding relationship with the advancing pads for forming continuous running length sleeve stock surrounding the pads; and, as the sleeve stock and the pads advance longitudinally, transversely cutting said sleeve stock at longitudinal positions intermediate said pads, thereby forming absorbent pads within sheaths having sheath extensions extending a sufficient distance beyond the ends of said pads for handling and/or securing said pads and sheaths.

Brief Description of the Drawings

Figure 1 shows a perspective view of the torso of an individual wearing incontinence care briefs according to the present invention.

Figure 2 shows a top view of the briefs of Figure 1, showing the appearance of the interior of the briefs when viewed through the waist opening.

Figure 3 is a sectional view taken along section line 3—3 of Figure 2 showing the placement of a moisture impervious panel in the briefs.

Figure 4 is a fragmentary sectional view taken along section line 4—4 of Figure 3, depicting a joint formed between the body of the briefs and the moisture impervious panel.

Figure 5 is a perspective view of a sheet of absorbent layer material useful in forming pads in accordance with the present invention.

Figure 6 is a perspective view of a composite useful for forming pads in accordance with the invention, and containing at least one absorptive layer similar to that shown in Figure 5.

Figure 7 is a fragmentary view of the end of a composite, for example the composite of Figure 6, which has been shaped to form undulations therein.

Figure 8 is a perspective view of a pad formed in accordance with the invention and comprising undulations, for example the undulations shown in Figure 7.

Figure 9 is an exploded view in perspective of layers of absorptive material forming a composite for an alternative to the form of pad disclosed in Figures 5 through 8.

Figure 10 is a top plan view of a pad formed using the composite of Figure 9, a portion of the pad being broken open to show the composite within.

Figure 11 is a bottom plan view of the pad of Figure 10 showing portions of an exterior wrapper being folded back to disclose the composite of Figure 9 within.

Figure 12 is an exploded view in perspective showing a composite design for forming a pad in accordance with the present invention.

Figure 13 is a perspective view showing a pad formed in a tri-fold configuration employing the composite of Figure 12.

Figure 14 is a bottom plan view of the pad of Figure 13 within a sheath.

Figure 15 is a top plan view of the pad of Figure 13 within a sheath.

Figure 16 is a partly schematic side elevation of the pad raw materials dispensing section of apparatus for carrying out the methods of the present invention.

Figure 17 is a side elevation of the aperture forming section of apparatus for carrying out the methods of the present invention.

Figure 18 is an enlarged perspective view of a portion of the apparatus of Figure 17 showing a cutter roller surface and absorbent material cut thereby.

Figure 18A is an enlarged portion of the surface of the cutting roller of Figure 18, in plan view, showing one of the cutting dies formed thereon for cutting hinge and flap members.

Figure 19 is an enlarged portion of the apparatus of Figure 17 showing a strip of absorptive layer material and the nip of a knock-out roller and mating pocket roller for displacing hinge and flap openings cut by the dies of Figures 18 and 18A.

Figure 20 is an enlarged portion of the apparatus of Figure 17 illustrating the strip with displaced hinge and flap members entering and passing through flattening rolls for further displacement.

Figure 21 is a partly schematic side elevation of the apparatus including a pad stock bending section which receives material from the apparatus of Figure 17 and a wrapping section.

Figure 22 is a sectional view taken along section line 22—22 in Figure 21 showing absorptive layers entering arrays of bending rails in the bending section.

Figure 23 is a sectional view taken along section lines 23—23 in Figure 21, depicting the layers of absorptive material deflecting into a zig-zag configuration in the bending section.

Figure 24 is a sectional view taken along section line 24—24 in Figure 21, illustrating a further development of the zig-zag configuration of Figure 23.

Figure 25 is a sectional view taken along section line 25—25 in Figure 21, showing a rotating disc array and cooperating grooved cylinder for assising in drawing the strip of absorptive material through the bending section of Figures 21—24.

Figure 25A is an enlarged portion of Figure 25 showing the tip of a disc in the aforementioned array and a mating groove in the cooperating cylinder.

Figure 26 is a sectional view taken along section line 26—26 in Figure 21 showing confining means which receive the layers of absorptive material in zig-zag configuration from the bending section.

Figure 27 is a sectional view taken along section line 27—27 in Figure 21 showing commencement of bending of a tissue wrap around the pad stock received in the bending section of the apparatus from the confining rollers of Figure 26.

Figure 28 is a sectional view taken along section line 28—28 in Figure 21 showing a further development of the bending shown in Figure 27.

Figure 29 is a sectional view taken along section line 29—29 in Figure 21 depicting the completion of the bending of Figure 28, so that the pad stock is encircled with the tissue wrap.

Figure 30 is a partly schematic side elevation of the pad cutting section and sheath material application section which receive wrapped pad stock from the apparatus of Figure 21.

Figure 31 is an enlarged portion of Figure 30 showing details of the nip of the cutting roller and cooperating cylinder which receive wrapped pad stock from the Figure 21 apparatus.

Figure 31A is an enlarged, fragment of Figure 31, showing a transverse cross-section of the profile of the cutting edge of the cutting roller.

Figure 32 is a sectional view taken along section line 32—32 in Figure 30, illustrating a sheath material dispenser which feeds sheath material in strip form to a translation roller in the sheath material application section of Figure 30.

Figure 33 is a sectional view taken along section line 33—33 in Figure 30, disclosing a stage in bending of the sheath material to encircle the wrapped pads which are being carried on the sheath material through the sheath material application section of Figure 30.

Figure 34 is a sectional view taken along section line 34—34 in Figure 30, showing completion of the bending depicted in Figure 33.

Figure 35 is an enlarged portion of Figure 30 showing inclined rollers for presenting the marginal edges of the sleeve material in an outward direction relative to the wrapped pad stock and feeding such edges into the belt-type fusion bonding apparatus of Figures 30 and 36.

Figure 36 is a partial sectional view, partially schematic, taken along section line 36—36 in Figure 30, showing details of the sheath material longitudinal heat-sealing apparatus.

Figure 37 is a partly schematic side elevation of the sleeve stock transverse heat-sealing and cutting section which receives pad stock surrounded by sleeve stock from the apparatus of Figure 30.

Figure 38 is an enlarged, partially broken-out portion of Figure 37 illustrating the nip of the sleeve transverse heat-sealing device and its cooperating cylinder, contacting and bonding the sleeve stock intermediate the ends of the pads.

Figure 39 is an enlarged, partially broken-out portion of Figure 37, showing a cutter roller and its cooperating cylinder for cutting the sleeve stock with pads therein to separate individual pads surrounded by sheath material with sheath extensions to facilitate securing or handling of the pads.

Detailed Description

The following is a detailed description of certain preferred embodiments of the invention. Like reference numerals identify like elements in each of the several figures of the accompanying drawings.

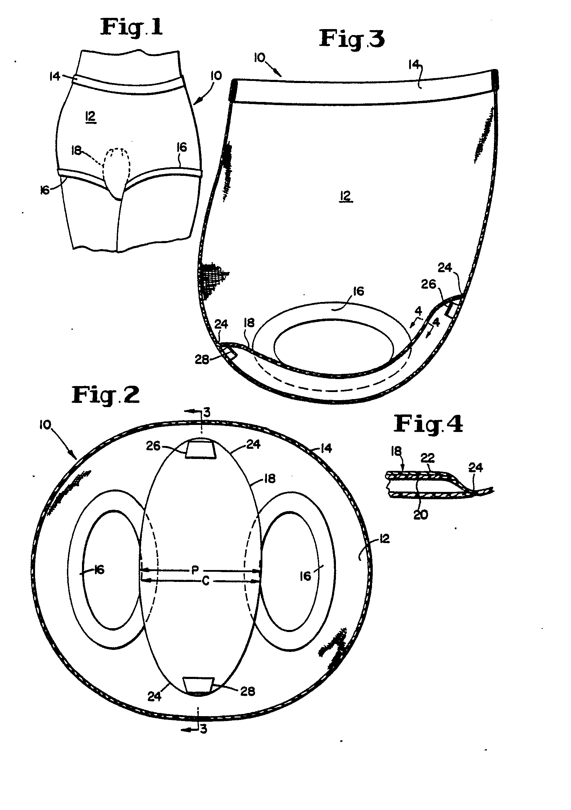

In Figure 1, a modified pair of briefs 10 is illustrated as it would appear on a person's body. Although briefs 10, as illustrated, preferably are of the type which extend from the person's upper thigh to the waist, those skilled in the art will understand that the invention also may be applied to other styles of pants such as bikini pants and the like. The body 12 of pants 10 may for example be made from a suitable knit or woven fabric made from such fibers as cotton, nylon, polyester or the like. At the waist, an elastic band or belt portion 14 is provided which includes posterior, side and front sections and may be approximately 1 to 2.5 cm in width and around the leg openings, no-elastic high stretch bands 16 are preferably provided, which also may be approximately 2.5 cm in width.

As shown in Figures 2 through 4, the interior of briefs 10 is provided with a holder portion that includes rearward, central and forward zones on which are mounted a moisture impervious laminated panel 18. This panel extends from a position rather low in the seat of the briefs, through the crotch or holder portion between the leg openings 16 and upward to a position rather high on the front of the briefs. As illustrated, panel 18 has an essentially elongated, oval geometry and comprises a lower layer 20 of a suitable knit or woven fibrous material such as nylon. A liquid impervious upper layer 22 of a thermoplastic or thermosetting polymeric film material such as vinyl is impregnated into or laminated to layer 20. As a result, panel 18 is somewhat stretchy but usually less stretchy than the body 12 of briefs 10. In a pair of briefs suitable for wearing by persons having waist sizes in the range of about 56 to 81 cm, the crotch width C shown in Figure 2 preferably is approximately 15.3 cm, including the leg bands. In such a case, panel 18 preferably has a width P of approximately 15.9 cm and a length of approximately 31 cm. Panel 18 is positioned so that as viewed in its longitudinal cross-section, the upper edges of its front and rear ends respectively appear approximately 21 cm and 10 cm above the bottom center portion of the crotch, measured along the profile of the panel.

When panel 18 has been positioned in this manner within a pair of briefs, it is joined to the briefs by any suitable means such as sewing or gluing and the like. Stretching of body 12 during such joinder is minimized to prevent puckering at the edges of panel 18. Preferably, panel 18 is attached using a peripheral heat joint 24 of approximately 0.32 to 0.48 cm (1/8 to 3/16) inch in width. Joint 24 may be formed using dielectric heat sealing equipment in which the periphery of panel 18 and a narrow portion of body 12 are compressed between dies and the temperature of thermoplastic layer 22 is elevated until the plastic flows through the cloth lower layer 20 of panel 18 into the interstices of the cloth forming the body 12 of the briefs to form a narrow band 24 of interlocked plastic and fabric, as shown in Figure 4.

After panel 18 has been joined to body 12 of briefs 10, a pair of elastic loops 26, 28 is stitched through panel 18 and body 12 at the front and rear edges of the panel, as shown in Figures 2 and 3, to act as retaining means for attachment of an incontinence pad to be worn within the briefs. The loops could also be attached to panel 18 before joint 24 is formed. To ease insertion of the sheath extensions which may be provided at each end of the incontinence pad, loops 26, 28 preferably are approximately 1.3 cm wide and 3.8 cm in length at their lower edges and 3.2 cm in length at their upper edges.

In general, the above described briefs may be used with any suitable pad including those presently known and those introduced hereafter. However, certain types of pads and features of pads, and combinations of such pads with briefs in accordance with the invention, constitute preferred embodiments which in themselves are considered to be inventions. Some of these will be discussed in greater detail hereinafter.

The pads provided in accordance with the present invention are characterized by the property of absorbing liquids. This includes anatomical fluids such as urine and non-anatomical fluids such as water and other polar and nonpolar fluids. However the primary and preferred application for these pads is in the absorption of urine and/or other anatomical fluids.

The absorptive pads used in the practice of the invention may include a single layer of liquid absorptive material. The preferred pads include, however, a plurality of layers of liquid absorptive material, referred to herein as "composites," which may include similar and/or dissimilar layers of absorptive and non-absorptive material, including layers which are and/or are not bonded to one another. The various layers may differ in their respective absorption rates and capacities.

A wide variety of materials are available, which are capable of being formed into layers and which are highly absorbent, i.e. capable of absorbing at least about 5, more particularly at least about 15 and most preferably at least about 35 times their own weight of clear water. Among the applicable materials are those which are capable of absorbing the same ratios of natural or synthetic urine, such as a 1% by weight saline (e.g. NaCl) solution. The uring capacities of these materials are usually less than, but may be equal to or greater than, their capacities for clear (i.e. pure) water. Examples of such materials may for example be found in British Patent Specification 1,515,768 and in U.S. Patents Nos. 3,890,974, 3,903,232, 3,935,099, 3,981,100, 3,985,616, 3,997,484, 4,069,821, 4,090,013, 4,093,765, 4,117,184, 4,144,886, 4,155,893 and 4,172,066, and in the following publications: B. Rånby and C. Rodehed, Polymer Bulletin, Vol. 5, 87, 1981; R. Mehrotra and B. Rånby, J. Appl. Pol. Sci., Vol. 21, pages 1647 and 3407, 1977 and Vol. 22, page 2991, 1978; and M. O. Weaver, et al, J. Appl. Pol. Sci., Vol. 15, page 3015, 1971 and Appl. Pol. Symposium 25, page 97, 1974, and in other documents mentioned in the foregoing publications and patents. Such highly absorbent materials may be in the form of particles, fibers, filaments, cellular solids, films and/or other forms, including both polymeric and non-polymeric material. When such materials are formed into sheets, certain of them are sometimes referred to as "hydrophilic paper," among which are included fibrous sheets that contain particles, fibers and/or films of polymeric or non-polymeric material that is water swellable or water soluble, such as for instance lightly cross-linked polyacrylates or polyacrylamides. Other examples include various modified starches, such as those having substantial proportions of their hydroxyl groups modified with grafted moieties which may be of short to long chain length and which provide reactive carboxyl groups in the resultant modified starch. A small proportion of these carboxyl groups may or may not be reacted with organic and/or inorganic di- or poly-functional cross-linking agents, and any desired additional proportion of these carboxyl groups including preferably major proportions thereof, may be reacted with organic and/or inorganic neutralizing materials, such as NaOH, KOH, amines and other bases. However, persons of ordinary skill in the art will readily recognize that a wide variety of other forms of absorptive layer material may be employed in the pad of the present invention. The most preferred material is that disclosed in U.S. Patent 4,117,184 issued September 26, 1978 to R. E. Erickson et al and assigned to the Dow Chemical Company, the entire disclosure of which is hereby incorporated herein by reference.

The preferred form of material disclosed in the aforementioned U.S. Patent 4,117,184 is a "sandwich" comprising a film of polymer that is water-swellable or gelable (i.e. forms a hydrated gel) sufficiently covered on at least two of its major surfaces with cellulosic fiber layers for imparting additional body or strength to the film and preventing blocking, as well as, reportedly, for protecting the polymer from environmental moisture. Preferably the entire thickness (i.e. all layers) of the sandwich are apertured. Some forms of this sandwich have the property of absorbing at least 50 times their original unswelled weight of clear water, with correspondingly lower ratios being exhibited with urine and other saline solutions. The preferred manner of using these highly absorbent sheet materials is to form them into a composite with one or more additional discrete layers of absorbent sheet material which are not part of the sandwich. According to a particularly preferred embodiment, plural sheets of the sandwich material are separated by sheets of lower liquid absorption capacity per unit of weight, such as tissue or wadding. According to a particularly preferred embodiment such discrete layers include one or more layers of paper which is not bonded to the sandwich and which is interleaved with sheets of the sandwich material in nesting relationship.

In a preferred embodiment of the pad comprising a combination of the absorptive sandwich material, as identified above, and one or more sheets of absorptive capacity lower than said sandwich, at least about 50%, preferably at least about 60%, and still more preferably from about 65% to about 80% of the total liquid absorptive capacity is present in the film of the water-swellable polymer.

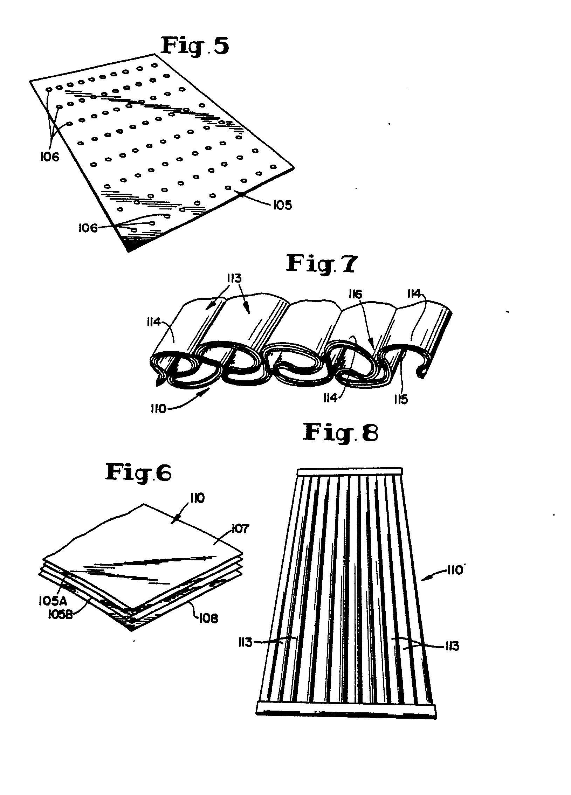

Figures 5 and 6 illustrate one form of composite which may be used in making a pad according to the invention. Figure 5 discloses a single sheet 105 of the sandwich material described in U.S. Patent 4,117,184, having the capacity to absorb about 35 or more times its own weight of natural or synthetic urine or other saline solutions. Said sandwich material has heretofore been commercially available in forms which included tiny openings or pinpricks, but was considered deficient in absorption rate for use as the primary absorption medium in urinary incontinence pads. The sheet 105 is provided with circular openings 106 distributed in a substantially uniform manner throughout its total area, extending between the upper and lower major surfaces of the sheet. The openings are formed by punching, so that the material encountered by the punch is completely removed from the sheet. In this example, the open area per opening is about $0.317 \ cm^2$, the number of openings per $cm^2$ of total sheet area is about 0.06, and the open area per unit area of said sheet is about 0.02.

As shown in Figure 6, a composite 110 is formed by placing two sheets 105A and 105B of the sandwich material 105 of Figure 5 between a pair of similarly sized sheets 107, 108 of ordinary tissue or wadding material. The sheets 105A and 105B in Figure 6 have the openings (not shown) which are depicted in Figure 5. The sheets 107, 105A, 105B and 108 may be secured together in any suitable fashion, such as by stitching, gluing, edge-taping or otherwise, and may, if desired, be provided with an outer layer of substantially water-proof or water-insoluble woven or non-woven fabric, preferably hydrophobic fabric. The composite shown in Figure 6 may be used in the planar mode shown therein, or may be used as a basis for forming a variety of different kinds of pads. According to a particularly preferred specie of the present invention, the pad is formed from absorptive layers which have been folded.

The foregoing is illustrated for example by Figures 7 and 8, which show the composite 110 of Figure 6 having been folded to form undulations 113. The word folded is used in a broad sense which does not require but does contemplate the possibility of creasing of the layers of material in the composite. The resultant undulations comprise concave and convex portions 115 and 114, respectively, the former defining ridges interspersed with furrows 116. As shown in Figure 8 the composite 110 with its undulations 113 may be secured at its ends in any suitable manner, such as for instance by taping, stitching, cementing or otherwise.

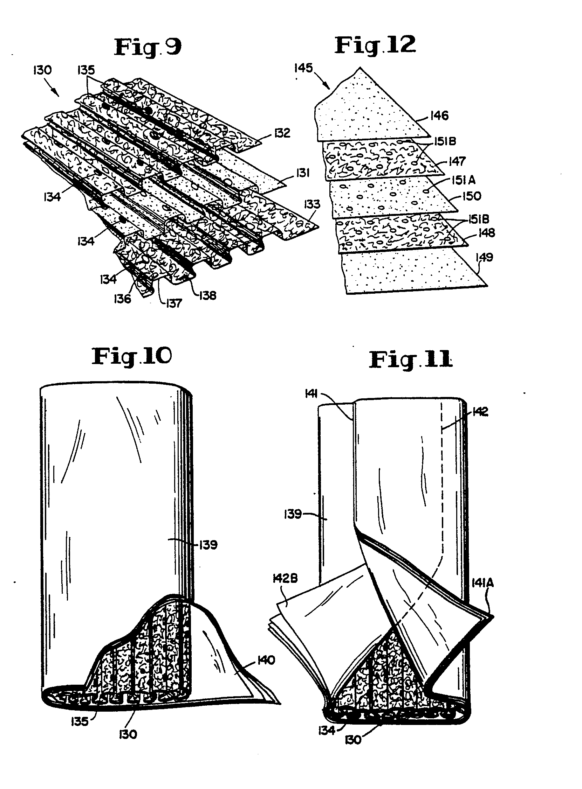

Figures 9—11 illustrate a still more preferable form of pad. In this embodiment a composite 130 is formed of outer layers 132 and 133 of absorptive polymeric film or hydrophilic paper, such as the sandwich material described above, separated by an intermediate layer 131 of ordinary tissue or wadding which is not included in the sandwich material, nor is it necessarily bonded or otherwise attached to the sandwich material. In this embodiment, all three sheets are provided with openings as above described and the openings 134 are preferentially positioned at or near the ridges formed by undulations 135 having convex and concave portions 136 and 137 and defining furrows 138 between them. Openings may also be preferentially formed at or near the bottoms of the furrows. Such positioning of the openings can be of assistance in imparting flexibility to the pad. To complete the pad composite 130 is wrapped with an outer wrap of two plies of tissue or wadding 139 as shown in the top plan view of Figure 10. A portion 140 of the tissue plies 139 is torn away and folded back (merely for purposes of illustration) to show composite 130 within. The outer wrap 139 has ends 141 and 142, the latter being folded over the former as shown in the bottom plan view of Figure 11. For purposes of illustration only, portions 141A and 142B of outer wrap 139 are folded back in Figure 11 to expose composite 130 and the openings 134. Note that the outer wrap 139, when closed, tends to obscure or conceal the openings and undulations, which may impart greater esthetic appeal to the pad.

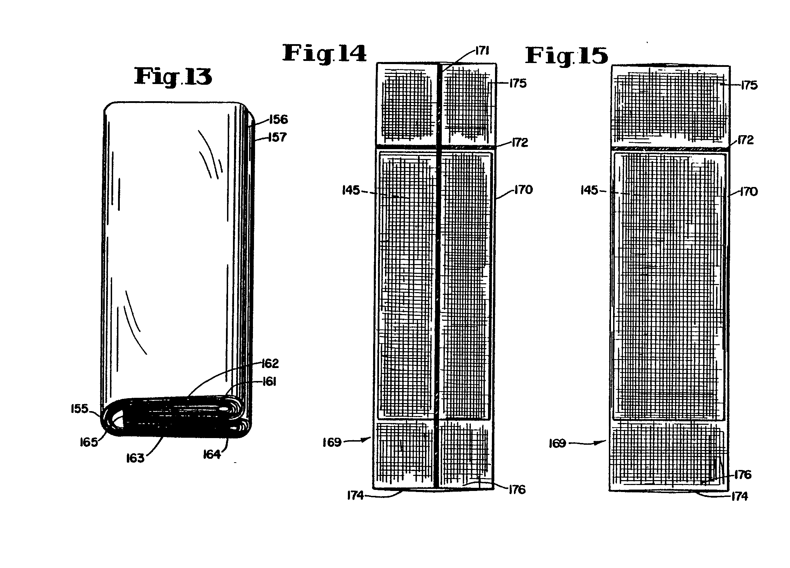

Assembly of another preferred form of pad is illustrated by Figures 12 and 13. A composite 145 is formed of a stack of five layers, including two layers 147 and 148 of hydrophilic paper separated and covered, above and below, with tissue or wadding layers 150, 146 and 149, respectively. Note that the three central layers 147, 148 and 150 are all provided with openings as above described, except that in this embodiment the openings 151A in the given absorptive layer, e.g. central tissue layer 150, are staggered, e.g. offset laterally or horizontally, in respect to openings 151B formed in an adjacent absorptive layer or layers, e.g. hydrophilic paper layers 147 and 148. The openings in adjacent layers may or may not coincide with one another, but it appears that a coincident arrangement may be beneficial from the standpoint of enhancing the absorption rate of the resultant composite. Composite 145 may then be folded into a tri-fold configuration as shown in Figure 13, along folds 155, 156 and 157, dividing it into four stacked quarters 161, 162, 163 and 164, and hiding the ends 165 of the sheets in the composite within the body of the resultant folded member. This pad, as well as the pads of Figures 5—6, 7—8 and 9—11 may be provided with woven or non-woven water-insoluble outer sheaths as above described, and these may be formed of hydrophilic and/or hydrophobic fibers, including polyesters and/or polyolefins, the latter being treated with wetting agents if desired.

Figures 14 and 15 contain an illustrative embodiment of a sheath 169 according to the invention. A light-weight open mesh fabric 170 of hydrophobic, seat-sealable polyolefin filaments is formed into a tubular member. This may be accomplished, for example, by folding a running length of said fabric along substantially its longitudinal center line to bring the longitudinal edges thereof into substantial juxtaposition and bonding said edges together by compressing and heating said material in a continuous or discontinuous elongated region 171 extending adjacent said edges and fusing the folded portions of the material together in said region. Both ends of the sheath may be left open for the discharge of wet pads. However, in the present preferred embodiment a transverse heat seal 172, uniting the walls of the flattened tubular sheath member 169, bars escape of the pad via one end of the sheath, while the other end may either be sealed shut, or, preferably, left as an open end 174 as shown. If end 174 is sealed shut, one may if desired provide an opening in another area of the sheath for pad removal, or provide a tear seal or other openable arrangement at end 174 or elsewhere in the sheath for the same purpose. In a preferred embodiment the tear seal or other openable arrangement will open in reponse to gentle shaking of the sheath when the pad is heavy from absorbed liquid.

If desired, a substantially water impervious sheet may constitute, or be positioned against the surface of, that portion of the sheath which will be adjacent the back of the pad in use. The water impervious sheet may also extend from the back up along the sides of the pad for assisting in confinement of liquid. For example, an elongated strip of porous woven or non-woven fabric suitable for forming the sheath and a narrower elongated strip of water impervious material can both be advanced longitudinally in face to face contact with their center-lines coincident and may be sealed to one another followed by the steps, described in the preceding paragraph, of forming a tube and sealing to complete a sheath.

When the sheath is longer than the pad, the extra length at each end provides extensions 175 and 176 which are useful in securing the pad in the brief and in handling wet pads. For example, the extensions 175 and 176, respectively, can be used with elastic loops 26, 28 (Figures 2 and 3) to secure the pad 145 in place at the forward and rearward zones of the briefs 10. Also see the description of the use of the sheath under "Advantages" below.

It should be understood that all or any portion of the above optional pad concepts may be applied individually or in combination with one another. These individual and combined pad options may furthermore be employed with or without the above described brief.

The above-mentioned briefs assist in insuring a correct forward/rearward positioning of the pads. However, wearers who do not require such assistance can use a form of pad which needs no special briefs. For example, if the undersurfaces of the pads are coated with pressure-sensitive adhesive which is capable of tenaciously gripping fabric and which may be covered with a tear-away protective strip to be removed when the pads are ready for use, the pads can then be supported in conventional fabric underpants. Such embodiments preferably include the above-mentioned sheath having a water-impervious backing sheet.

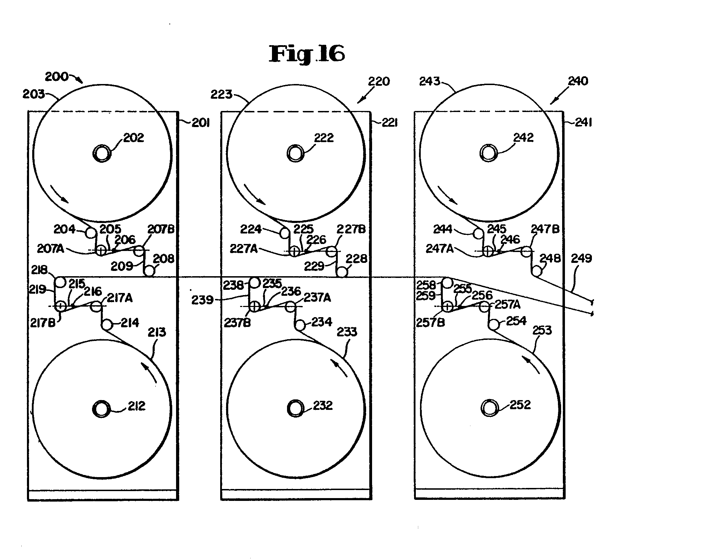

For purposes of illustrating the method, the pad will include layers of highly absorbent material having between them a two-ply layer of tissue which performs a wicking action. Figures 16 through 39 illustrate the method of the present invention and suitable apparatus for carrying out said method on a continuous basis with the absorptive material and tissue being supplied in the form of strips wound into rolls.

The apparatus may include for example a tissue dispenser 200 and first and second absorptive strip material dispensers 220 and 240 positioned in sequence, with the tissue dispenser upstream of the absorptive strip material dispensers. Preferably each of the dispensers is provided with means for feeding strip material from either of two unwinding means whereby strip material may be dispensed from one of the unwinding means while a spent roll of strip material is replaced with a fresh roll on the other unwinding means. Appropriate placement of the unwinding means enables commencement of unwinding from the standby roll of strip material when the roll that is currently being unwound is exhausted.

Thus, the tissue and absorptive strip material dispensers of Figure 16 are each provided with frames 201, 221 and 241, the upper unwinding means of which include first expandable mandrels 202, 222 and 242. These mandrels include means which can be selectively expanded or retracted to grip or release the core of a roll of tissue or absorptive strip material. Webs which unwind from the several rolls 203, 223 and 243 pass over guide rollers 204, 224 and 244 to tension sensing means. The webs pass respectively around sensor rollers 207A, 227A and 247A, pass diagonally across the respective tension sensor beams 205, 225 and 245 and around the remaining sensor rollers 207B, 227B and 247B. The respective tension sensor beams are biased around their respective pivots 206, 226 and 246 to pick up any slack which may develop in the webs, and are connected to linkages and brake mechanisms (not shown) which brake the respective mandrels 202, 222 and 242 sufficiently to inhibit development of slack in the webs and prevent over-running of the rolls 203, 223 and 243. Downstream of the respective tension sensing means are alignment rollers 208, 228 and 248 which direct the respective webs 209, 229 and 249 along the desired feeding paths.

Each of the dispensers 200, 220 and 240 includes a second unwinding means having components similar in nature and function to the first set of unwinding means described above. These include, respectively, second expandable mandrels 212, 232 and 252 for unwinding rolls 213, 233 and 253 around guide rollers 214, 234 and 254, across tension sensor beams 215, 235 and 255 provided with pivots 216, 236 and 256 as well as sensor rollers 217A, 217B; 237A, 237B; and 257A, 257B, and further around alignment rollers 218, 238 and 258 to guide the webs 219, 239 and 259 into the desired web feeding paths.

In operation, assuming that tissue is being fed from roll 203 on mandrel 202, a standby roll of

**0 062 495**

tissue 213 is installed on mandrel 212. When roll 203 is exhausted, the feeding of roll 213 is commenced. The absorptive strip material may be fed from either of the upper mandrels 222 or 242 and from either of the lower mandrels 232 or 252. The unused mandrels in dispensers 220 and 240 are provided with standby rolls. When the upper absorptive material roll 223 or 243 is nearing exhaustion, feeding from the other upper roll is commenced. A similar routine is used to commence the feeding of the standby roll of absorptive material on one of the lower mandrels when the operational lower roll is exhausted. The depicted dispensers may of course be replaced with ones have the capability of commencing the feeding of stand-by rolls on the fly.

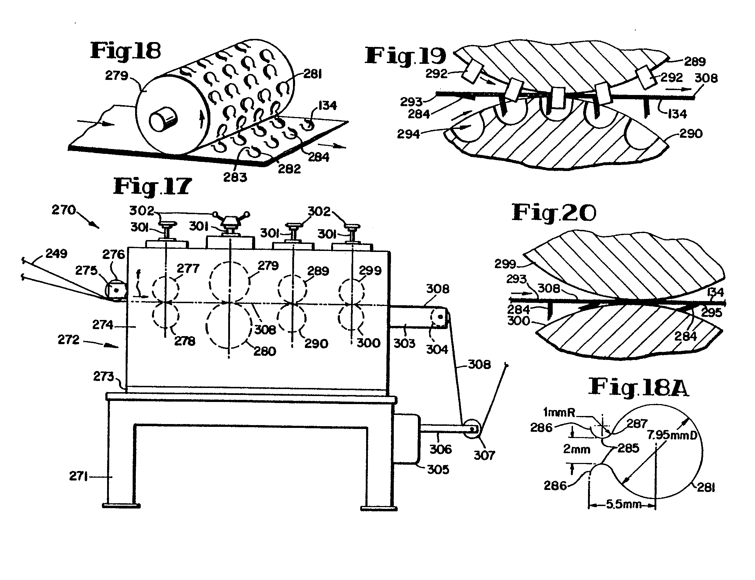

According to a preferred embodiment the apparatus of the invention includes an aperture forming section including a cutter unit 270. The latter may for example have a frame 272 mounted on table 271 and comprising a base 273 upon which are erected two parallel plates, only one of which, plate 274, is visible in Figure 17 in side elevation. The second parallel plate is directly behind the one shown and is spaced apart therefrom to provide space between them for mounting various processing rollers and cylinders, the latter having axles (not shown) journalled in bearings (not shown) mounted in the parallel plates. The axes of the respective rollers and cylinders are perpendicular to the plates, perpendicular to the direction of advancement of the material processed therein and parallel to the plane in which such material moves. The aperture forming section receives a composite comprising the strips of absorptive material separated by an intermediate layer of tissue, such composite being guided into the aperture forming section by an idler roller 276 mounted on frame extensions 275, one of which is visible in the drawing. The material then passes to top and bottom infeed drive rollers 277 and 278 from which it progresses to a cutting roller 279 having raised cutting projections to form the desired apertures, which projections bear against the top surfaces of, and pierce through, the composite, which is supported from beneath by a hardened plain cylinder 280.

As shown in greater detail in Figure 18, constituting a fragmentary enlarged illustration of a portion of cutting roller 279 and the resultant material, the apertures or openings may optionally be formed in such a way as to cut through the material of the composite along a portion, but not all, of the perimeter of the respective openings, thereby forming in said composite hinge and flap members. As shown in Figure 18 and in an enlarged portion of Figure 18 shown in Figure 18A, the peripheral surface of cutting roller 279 is provided with a regular pattern of cutting dies 281 having their cutting edges arranged in a configuration which will cut the absorptive layer material(s) along lines that partly but not completely surround the area of the intended openings 134. A first portion 282 of the perimeter of each opening is cut, and a second portion 283 of the perimeter is not cut. These uncut portions 283 form hinge members, while the cut portions define not only portions of the openings, but also the perimeters of flap members 284.

While Figure 18A represents the outline for the cutting edges of dies 281 on cutter rollers 279, it also represents the cutting line formed in the work material. As the figure indicates, the die and its corresponding cutting lines include gentle, smooth curves 285 which respectively form and curve past the edges of the hinge portions to ends 286 which diverge from one another. This can assist in inhibiting tear propagation in the layer(s) in and/or adjacent the hinge members. In this embodiment the gently, smoothly curved portions of the dies and cutting lines also include curved transitions 287 from the openings per se to positions alongside the hinge portions.

When hinge and flap members are formed, the flap members should be displaced, in one or more steps, so that they do not block the openings from they are formed when the composite is formed into pad stock. Coincident hinge and flap type openings in the several layers of a moving composite, if displaced outwardly together, can cooperate to bind the layers together and promote coordinated movement of the several layers. Best results are attained when the hinges are formed with lesser width, measured along the hinge bending lines, than the maximum widths of the openings, measured parallel to said bending lines, and the hinge members are formed by cutting lines which curve in the above-described manner. The above-mentioned displacement of flap members may be accomplished in one or more steps.

As shown in Figures 17 and 19, this is preferably accomplished in two steps employing a knock-out roller 289 driven in synchronization with cutting roller 279 and having spikes 292 or other projections positioned so that they will contact the composite 293 passing from roller/ cylinder combination 279/280 in registry with the respective flap members 284 and displace said flap members outwardly, in this case downwardly, relative to the composite and openings 134. The displaced flaps temporarily enter small pits 294 in a pocket roller 290 which is mated to knock-out roller 279 as shown in Figure 19.

In an optional second displacement step shown in Figure 20, the composite 293 and the flap members 284 are rolled between flattening cylinders 299 and 300 whereby the flap members are displaced inward, in this case upward, relative to the composite. Thus the flap members can be flattened against portions of the composite adjacent the respective hinges 295 while leaving the respective openings 134 substantially open, i.e. not blocked by the flap members. When the previously described first displacement (and second displacement, if any) are performed in such a manner that the flaps 284 are left extending away from the composite 293 from which they are formed, and the layer or layers are eventually formed into an undulating pattern which is retained in the resulting pad, these flaps can act as spacers between adjacent undulations for promoting a high absorption rate. After the displacement operation(s), as shown in Figure 17, the apertured composite 308 then passes over outfeed idler roller 304, rotatably mounted on fixed downstream frame extension 303, and traverses the idler roller 307 of constant tension device 305 having a horizontal arm 306 which translates roller 307 upwardly and downwardly to maintain constant tension in web 308.

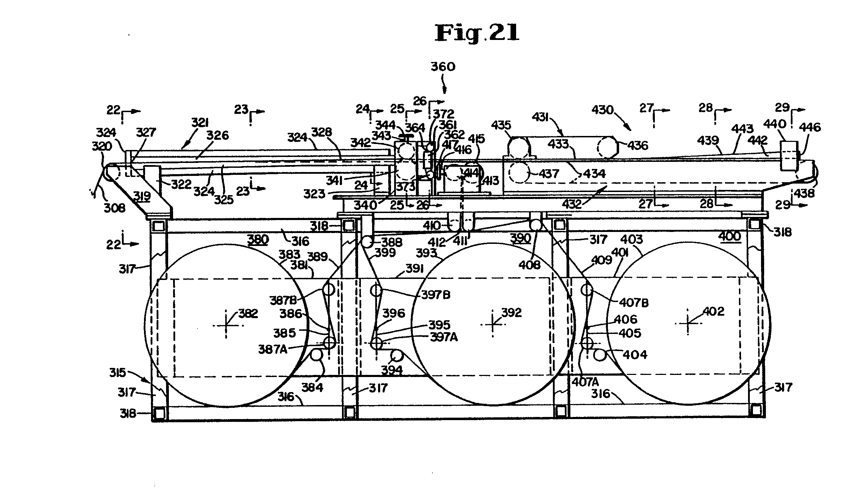

As shown in Figure 21 the preferred apparatus for performing the method of the present invention includes a pad stock folding section 321 and an absorbent fiber wrapping section 430, these elements being mounted in a common frame 315 having longitudinal stringers 316, uprights 317, transverse members 318 and a frame extension 319. The apertured web 308 from the cutter unit 270 passes over infeed idler roller 320 rotatably mounted in the frame extension 319 and immediately passes into folder 321. The latter includes frame members 324 supported by first and second legs 322, 323. Within frame 324 are distributed a first array of upwardly directed rails 325 in the lower portion of frame 324 beneath web 308. A second array of downwardly directed rails 326 is positioned in the upper portion of frame 324 above web 308. The rounded infeed ends 327 of arrays 325 and 326 facilitate entry of the web with dependent flap members 284.

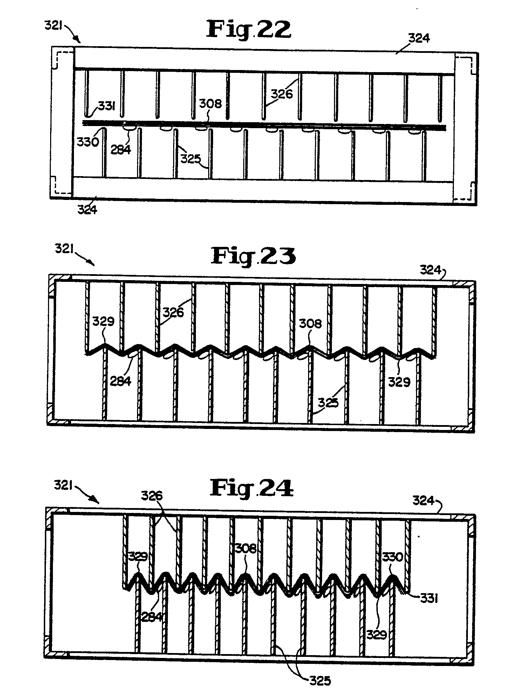

Rails 325 in the lower array extend in the general direction in which the web moves but, as shown in Figures 22—24, are spaced laterally from one another. The rails 326 extend in the same longitudinal direction and are also laterally spaced from one another with the rails 326 being positioned above or within the space between the rails 325. For simplicity, the longitudinal convergence of rails 325 and 326 has been eliminated from the background in Figures 22—24. Scanning the respective arrays from left to right in cross section, it will be seen that the upper and lower rails 326 and 325 alternate laterally across the structure. While the upper surfaces 330 of rails 325 are all in a common plane and the lower surfaces 331 of rails 326 are also in a common plane, these planes are not parallel. Thus there is a small vertical spacing between said planes at the infeed end of folder 321. These planes intersect with one another intermediate the infeed ends 327 and output ends 328 so that the lower surfaces 331 of rails 326 are below the upper surfaces 330 of rails 325 at their output ends. Also, as the rails extend from their infeed ends downstream towards their output ends 328 they converge laterally. By virtue of the intersection of the above mentioned planes and the lateral convergence of the two arrays of rails, the advancement of web 308 and dependent flaps 284 longitudinally through the folder causes a plurality of bends 329 to form in the web running lengthwise thereof and spaced at intervals across the width of the web between flaps 284. In such manner the web 308 is formed into an elongated pad stock which is of greater thickness and narrower width, in which the web has a zig-zag configuration and in which the flaps can act as spacers between the undulations of the zig-zag pattern.

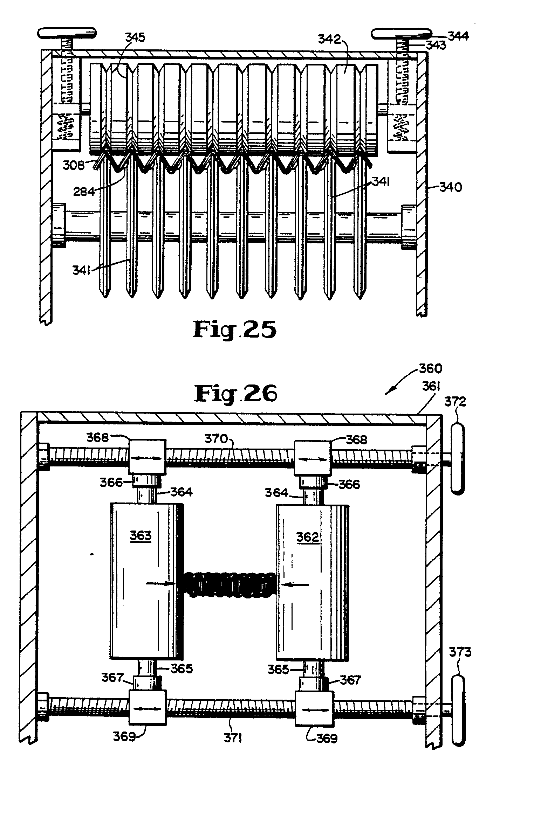

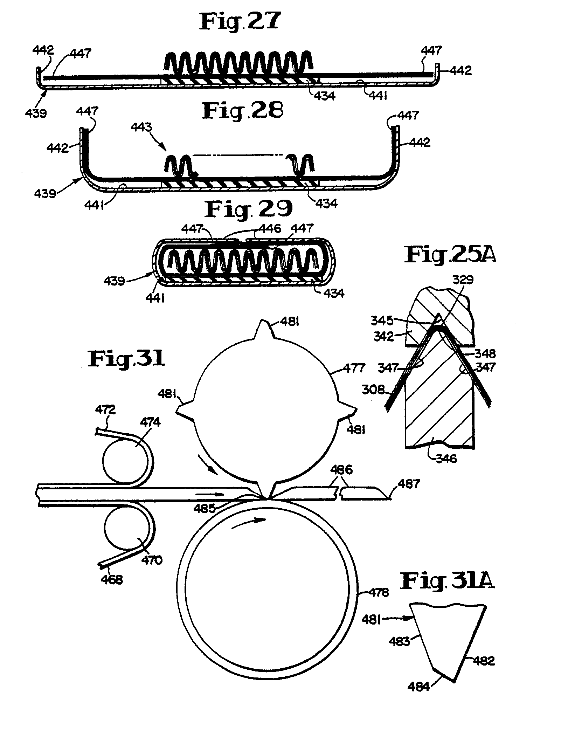

Referring again to Figure 21, the pad stock comprising folded web 308 is drawn out of the folder section 321 by a rotating disc-cylinder assembly adjacent the output ends 328 of the rails 325 and 326. This assembly comprises stanchions 340, which are visible in Figures 21 and 25, and between which is secured a driven, rotating array of discs 341 in which the respective discs are mounted with their apexes at substantially the same elevation as the output ends 328 of the upwardly directed or lower rails 325, the discs also being spaced apart laterally by the same distances as, and also aligned with, the corresponding output ends 328 of these rails. An elastomer-surfaced cylinder 342 positioned above disc array 341 maintains contact with the upper outer surfaces of the upper bends in the pad stock, and urges the inner surfaces of such bends into driving contact with the edges of the respective discs in array 341. The pressure of cylinder 342 against the bends in the pad stock and the disc array 341 can be set with adjusting screw 343 and handle 344.

As shown in part in Figure 25 and in greater detail in Figure 25A, an enlarged portion of Figure 25, it is recommended that elastomer-surfaced cylinder 342 be provided with grooves 345 in alignment with discs 341. Figure 25A shows that the peripheries 346 of the discs include chamfered or convergent surfaces 347 which intersect in curved surfaces or radii 348, as viewed in a plane which includes the axes of rotation of the discs. These radii inhibit cutting of the web 308 at the extreme upper portions of the bends 329 which are compressed between and frictionally engaged by the convergent disc peripheral surfaces 347 and the groove walls 348. This grooved arrangement has proven to be of significant assistance in controlling machine gain relative to the travelling web 308 at the downstream end of the folding section 321 of Figures 21—24. The appropriate amount of traction between discs 341 and webs 308 with varying stiffness and frictional characteristics can be obtained by varying the included angle and depth of grooves 345.

Downstream of the disc arrays 341 and cylinder 342 the width and lateral position of the pad stock can, as shown in Figs. 21 and 26, be controlled by lateral support means 360 comprising subframe 361 mounted on the frame 315 and supporting a first roller 362 and a second roller 363, these rollers being mounted with their axes vertical so that they laterally confine or compress the pad stock between them. These rollers may be mounted in such a way as to provide for adjustment of their lateral spacing. For example, the shaft means 364, 365 supporting the rollers 362, 363 in a vertical position may be rotatably mounted in bearings 366, 367 secured to blocks 368, 369 which are laterally shiftable in subframe 361. The upper and lower blocks, indirectly supporting both ends of the left and right rollers, are each threadedly engaged with one of two oppositely pitched thread sections on adjusting screws 370 and 371 having handles 372 and 373. Thus, adjustment of screws 370, 371 controls the lateral gap between rollers 362, 363.

After the above mentioned pad stock has been formed, it is advanced longitudinally and brought into contact with at least one strip of liquid permeable material such as absorbent fiber material which is wider than the pad stock. The pad stock and fiber material strip are then caused to advance together longitudinally while bending the strip along longitudinal lines for encircling the pad stock, as viewed in transverse cross section. Thus a wrap of fiber material is formed around the pad stock. As shown in Figure 21, this may be accomplished with the aid of fiber material dispenser 380, 390 and 400 operating in combination with folder 321 and a wrapping section 430. These dispensers are similar to the dispensers 200, 220 and 240 of Figure 16, except that they each include only one unwinding means. Thus the respective dispensers 380, 390 and 400 include frame members 381, 391 and 401 and expandable mandrels 382, 392, and 402 which support rolls 383, 393 and 403 of fiber material for rotation and unwinding. As the fiber material unwinds it passes over guide rollers 384, 394 and 404 to the sensor rollers 387A, 387B; 397A, 397B and 407A, 407B of tension sensor beams 385, 395 and 405 having pivots 386, 396 and 406 which function in the manner described above. From these are delivered fiber webs 389, 399 and 409 which pass over guide rollers 388 and 408 along the desired feeding paths.

After passing guide rollers 388 and 408, the fiber wrap webs enter a gap 412 situated between first and second pinch point rollers 410, 411. The webs then pass to fiber material infeed rollers 414, 415 mounted on subframe 413 and adjustable by means of compression adjustment screw 416 and handle 417.

In the preferred mode of operation, any two of the mandrels 383, 392 and 402 are employed to unwind two operational rolls of two ply fiber material. Thus a pair of webs, drawn from any two of the three mandrels, is caused to pass between pinch point rollers 410, 411 to the infeed rollers 414, 415 and from thence into contact with the underside of the pad stock. While two of the mandrels are unwinding operational rolls, the third mandrel may be loaded with a standby roll. When one of the operational rolls has run out, the unwinding of the standby roll may be commenced.

The pad stock and the wider underlying double layer of the fiber material enter wrapping section 430 with the aid of pad stock and fiber material belt feeders, such as upper feeder 431 and a longer lower feeder 432. The upper feeder includes a belt 433 held in position by upper infeed belt roller 435 and upper outfeed belt roller 436 so that the lower driving surface of belt 433 contacts the upper surface of the pad stock. The belt 434 is held in position by lower infeed belt roller 437 and lower outfeed belt roller 438 with the upper driving surface of belt 434 in contact with the underside of the fiber webs. The belt feeders 431, 432 are driven so as to transport the pad stock and fiber webs from left to right towards the channel 439 which is secured to the frame of lower belt feeder 432 and is further supported at its downstream portion 446 by a channel frame member 440. Upper belt feeder 431 terminates a short distance upstream of channel 439 while the longer belt feeder 432 extends beyond the downstream end of channel 439 with its belt 434 inside channel 439 along its inner surface 441. See the detailed cross sectional views of Figures 27, 28 and 29, in which the channel 439 has upturned side walls 442 which converge laterally and progressively increase in height in the downstream direction, thereby diminishing the width of the channel inner surface 441 and causing the marginal edges 447 of the fiber material to turn upward as the fiber material passes through the longitudinal mid-portion 443 of the channel. As the side walls 442 converge still further, approaching channel downstream portion 446 (Figures 21 and 29), they are bent inward more or less horizontally to form top walls 446 extending into downstream portion 446 and causing the marginal edges 447 of the fiber material to overlap one another. This causes the fiber material to fully enclose or encircle the pad stock as viewed in transverse cross section, thereby forming a wrap of fiber material around the pad stock.

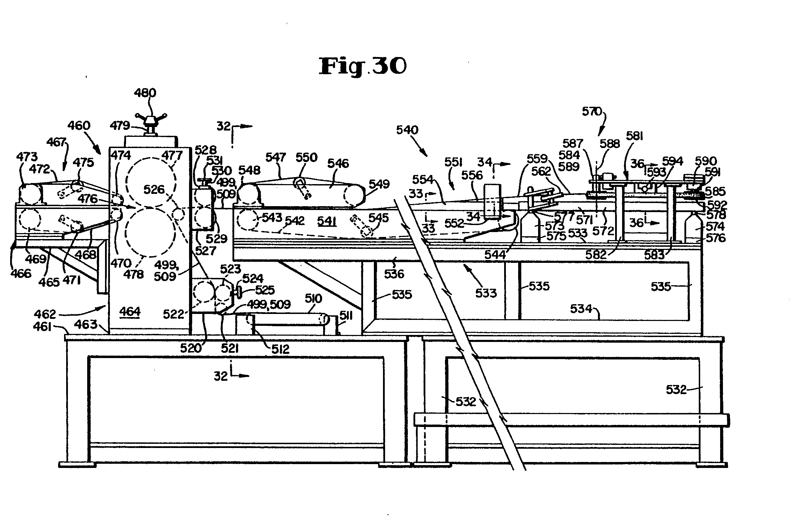

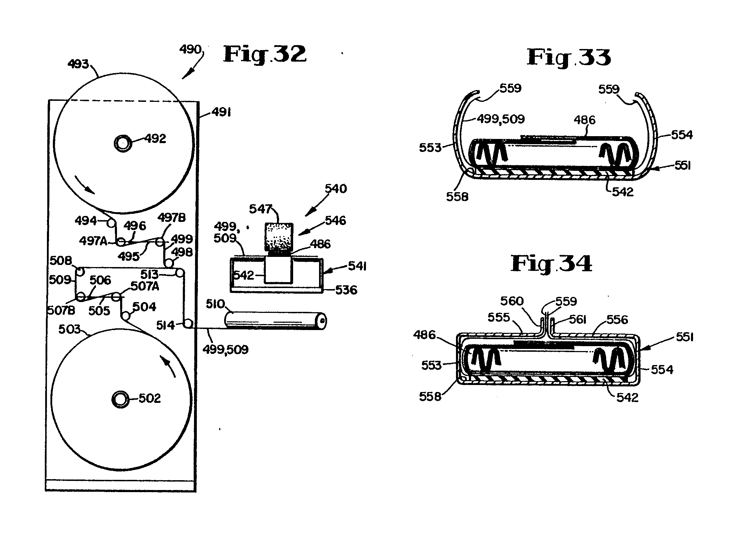

According to one embodiment of the invention the pad stock and fabric wrap are cut transversely in a common cutting step to form pads of predetermined length therefrom. According to the present embodiment of the invention, as shown in Figures 30, 31 and 31A, such cutting can be accomplished while the pad stock and fiber web are advancing longitudinally, pads of predetermined length being cut therefrom. As indicated in Figure 30, this may be accomplished for example by pad cutting section 460 erected on table 461 and having a frame 462 of parallel plates 464 mounted on base 463. The plates 464, only one of which is visible in side elevation in Figure 30, are aligned generally parallel to the direction of material flow and are spaced apart a sufficient distance for the passage of processed material and for the mounting of a cutting roller and corresponding cylinder between the plates. An upstream frame extension 465 supports belt feeders 466 and 467 which receive the wrapped pad stock from wrapping section 430 in Figures 21 and 29. The surface of the wrapped pad stock is contacted by the upper surface of lower belt 468, supported, driven and tensioned by lower infeed roller 469, lower outfeed roller 470 and tension roller 471 in lower belt feeder 466. The upper surface of the wrapped pad stock is engaged by the lower drive surface of upper blet 472, supported, driven and tensioned by upper infeed roller 473, upper outfeed roller 474 and tension roller 475 in upper belt feeder 467. Belt feeders 466 and 467, driving in unison, deliver the wrapped pad stock to the nip 476 of a raised edge cutting roller 477 having raised cutting edges distributed at angularly spaced intervals about its periphery, the peripheral distance between such cutting edges corresponding with the desired pad length.

As shown in the enlarged views of Figures 31 and 31A, the cutting edges 481 are situated at an appropriate radial distance from the center of rotation of roller 477 so that they bear against the smooth surface of a polished hardened steel plain cylinder 478 which supports the raised pad stock during cutting while rotating in unison with, i.e. at the same peripheral speed as, cutting roller 477. The cutting pressure exerted by cutting roller 477 can be adjusted by means of compression adjusting screw 479 with handle 480. Preferably, cutting edges 481 include generally upright but downwardly convergent forward and rearward surfaces 482 and 483 which both intersect with a narrow compression surface 484 that is inclined downwardly in the direction of movement of the work through the rollers 447, 448 and is more nearly horizontal than vertical. The intersection of surfaces 482 and 484 forms a cutting edge and the surface 484 applies compression to a narrow band of material 485 adjacent the trailing edge of each cut. This causes collapsed and overlapping undulations and/or other layers in the pad stock to cling to one another with sufficient adherence to form a coherent leading edge 487 at the front of each pad 386 which retains substantial structural integrity during subsequent advancement of the resultant cut pad sections. When the cut sections are advanced longitudinally into contact with sheath material while the latter is advancing longitudinally at an accelerated rate, these coherent leading edges help resist fraying of the pads 486 and/or jamming of the equipment.

It should be understood that the lengths of wrapped pad stock can themselves function as absorbent pads. However, for reasons explained above it is useful to provide such pads with an outer sheath, such as for instance a sheath material which is permeable to the liquid to be absorbed and yet it retains its structural integrity when moistened with the liquid. This sheath can perform optional but useful protective and/or handling functions described above. Such protective functions include, for example, inhibiting the wicking of moisture from a wet pad with the sheath to the outer surface of the sheath which would likely be in contact with the wearer's skin in the case of an incontinence care pad. The handling functions include providing a length of material extending generally longitudinally from each end of the pad for use in securing the pad within a garment and/or carrying the used pad from the garment to a disposal point such as a toilet. Depending on whether protective and/or handling functions are needed in respect to a particular absorbent pad product it may or may not be desirable to apply a sheath member to the pads. If such sheath member is applied it may or may not be desirable to provide a significant increment of additional length in the sleeve, as compared to the length of the pad, to facilitate handling. The formation of a sheath with or without extensions of substantial length can be performed by the remaining portions of the machine shown in in Figures 32 through 39 and comprising a sheath material application section, a sheath material longitudinal sealing section and a cutting section to be described in greater detail below.

The sheath material application section, shown in Figure 32, includes a sheath material dispenser 490 similar to the tissue dispenser 200 of Figure 16. It is mounted to one side of the material flow path through the main portion of the apparatus, with its frame 491 perpendicular, and its mandrels 492, 502 parallel, to the direction of travel of material through said flow path. Thus, as in the previously described dispenser, the mandrels 492 and 502 support standby and operational rolls 493 and 503, but in this case the rolls are rolls of the sheath material. The latter passes around guide roller 494 or 504 to the sensor rollers 497A, 497B; 507A, 507B of the tension sensor beams 495, 505 with pivots 496, 506 and from thence to alignment rollers 498, 508 which direct the webs 499, 509 of sleeve material via further guide rollers 513 and 514 to the underside of a translation roller 510 (Figures 30 and 32) supported by stanchions 511 and 512 on table 461 (not shown in Figure 32). Translation roller 510 is mounted with its rotational axis in a horizontal plane and at an angle of 45° to the direction of travel of the pad stock and wrapped pads. Sheath material passes from dispenser 490 in a direction perpendicular to the main material flow path and in the process of passing around translation roller 510 is redirected so that as it leaves the top of roller 510 it then moves in a direction directly opposite to the flow of wrapped pads but at a lower elevation than the pads. As best shown in Figure 30, after the web of sheath material 499, 509 is turned upstream by roller 510, it passes around a turning roller 521 mounted in frame extensions 520 secured to the parallel plates 464 of pad cutting section 460. From roller 521 the material progresses to the nip of first and second sheath material infeed rollers 522, 523 driven at the same peripheral speed. The compression between rollers 522 and 523 may be adjusted by compression adjustment screw 524 with handle 525. The infeed rollers draw the web from translation roller 510 and deliver it to a turning and alignment roller 526 journalled in bearings (not shown) mounted in the parallel plates 464. As the web clears roller 526 it is at the appropriate elevation to receive the cut-to-length pads discharged from cutting roller 477 and cylinder 478, thus becoming a support for the pads as they travel further downstream in the process.

Depending on how long one wishes to make the sheath, as compared to the pad length, thereby controlling the length of the sheath extensions, the web of sheath material will be caused to travel at a somewhat greater velocity than the velocity of the pads ejected by cutting roller 477 and cylinder 478. The greater speed of the sheath material web causes the pads to advance longitudinally at an accelerated rate relative to the rate of advancement of the pad stock and outer wrap during cutting, thereby causing the pads to travel longitudinally on the sheath material web with a predetermined space therebetween. By adjusting the difference in said rates one can adjust the lengths of the sheath extensions to be formed subsequently, such lengths varying for example from the minimum distance required to seal the sheath material at one or both ends of the pad, to the maximum useful length of sheath extension which might be employed for securing the sheaths and pads in a garment and/or transporting them from the garment to a disposal receptacle. According to one embodiment the pads are caused to travel longitudinally on the sheath material with a predetermined space between the pads which is about twice the combined length of the sheath extensions which are to be formed. The advancement of the pads and sheath material web is assisted in part by upper and lower pad/sheath material drive rollers 528/529 mounted for rotation in second frame extensions 527 secured to parallel plates 464 of the pad cutting section 460. The pressure developed between rollers 528 and 529 may be regulated by compression adjusting screw 520 with handle 531.

According to an optical embodiment of the invention the sheath material is bent along a longitudinal line or lines for encircling the pads. In the present preferred embodiment such bending is accomplished while the pads are being carried upon and advanced longitudinally with the sheath material. As shown in Figure 30, the preferred apparatus for performing this operation is mounted on a sheath material wrap and seal subframe 533 including lower stringer 534, uprights 535 and upper stringer 536 upon which is mounted the feeder/sheath wrapper section 540.

As may be seen in Figure 30 and in Figure 32, from which the equipment supports and background detail have been omitted, the cut-to-length pads supported on the wider underlying layer of sheath material enters sheath material application section 540 with the aid of pad and sheath material belt feeders, such as upper feeder 546 and a longer lower feeder 541. The upper feeder 546 includes a belt 547 positioned, driven and tensioned by upper infeed roller 548, upper outfeed roller 549 and tension roller 550, so that the lower driving surface of belt 547 contacts the upper surfaces of the pads 486. The belt 542 is positioned, driven and tensioned by lower infeed roller 543, lower outfeed roller 544 and tension roller 545 with the upper driving surface of belt 542 in contact with the underside of the sheath material 499, 509. The belt feeders 541, 546 are driven so as to transport the pads and sheath material from left to right (as viewed in Figure 30) towards the channel 551 which is secured to the frame of lower belt feeder 541 and is further supported at its downstream portion by a frame member 552. This channel assists in wrapping the sheath material about the pads.

Referring now to Figure 30 and cross-sectional views 33 and 34, the shorter upper belt feeder 546 terminates a short distance upstream of channel 551, while the longer, lower belt feeder 541 extends beyond the downstream end of channel 551 and belt 542 extends inside channel 551 along its inner surface. Upstream of Figure 33 channel has left and right lateral fences 553, 554. These converge laterally in the downstream direction, thereby diminishing the width of the channel floor 558 and causing the marginal edges 559 of the sheath material 499, 509 to turn upward as it passes through the longitudinal mid-portion of channel 551 as shown in Figure 33. As the upper edges of these fences approach the channel downstream portion which is shown in cross-section in Figure 34, they are lengthened and bent to a horizontal configuration to form left and right inward projections 555, 556, with upturned lips 560, 561 at their inner extremities. These cause the marginal edges 559 of the sleeve material to project outwardly relative to the nearest major surface of the pad stock or pads 486, in this case turning generally upwardly, while also enclosing or encircling the pads as viewed in transverse cross section, thereby forming a wrap of sleeve material around the pads.

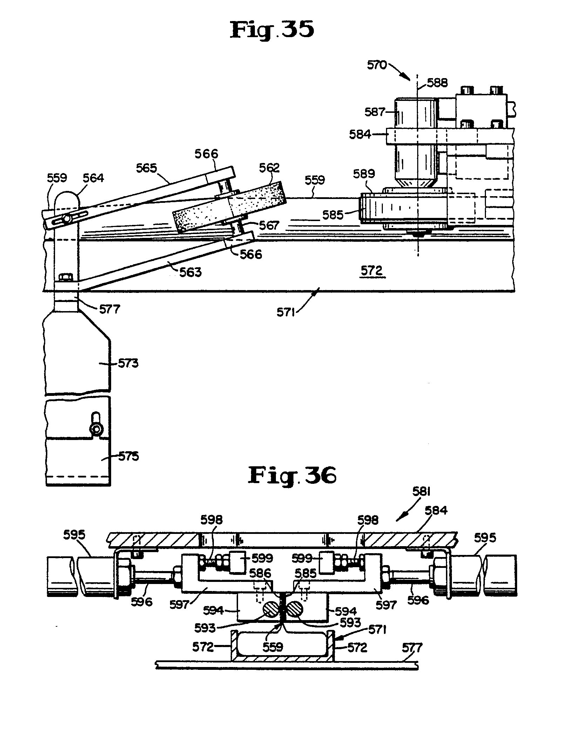

As shown in Figure 30, and in greater detail in its enlarged portion illustrated in Figure 35, the outward projection of the longitudinally advancing marginal edges can be assisted or caused to occur by advancing said edges through the nip of a pair of abutting, driven or non-driven rotatable rollers having their axes of rotation inclined from the vertical in the upstream direction. If the pad stock is travelling generally horizontally with the sheath material marginal edges above it, and projecting upwardly, and with the inclined axis rollers being situated above the pad stock, such rollers will cause lifting of the marginal edges and are preferably employed to feed the marginal edges from channel 551 into sealer section 570. In the present embodiment, the rollers 562 (only one of which is visible in Figures 30 and 35) are supported by the same base 575, stanchion 573 and transverse member 577 that support the sealer section 570, to be described in greater detail below. The second roller abuts with and is directly behind the one shown, on the opposite side of the work. The two rollers have left-and right-hand mounting brackets of similar design, including lower support arms 563 and posts 564 secured to transverse member 577, as well as upper support arms 565 secured to posts 564. Support arms 563 and 565 have a moderate upward inclination in the downstream direction and have their downstream extremities bearings 566 rotatably supporting the shaft 567 with its axis of rotation inclined in the above-described manner. The rollers 562 are rotated on these shafts by the work which frictionally engages the rollers as it passes between them. Thus, the sheath material lateral edges 559, presented upwardly upon the discharge from channel 551, rise from left to right as they progress from channel 551 toward the rollers 562. The inclined rollers discharge the sheath material 499, 509 with the pads 486 within (not shown) and with the marginal edges 559 at the proper elevation for bonding, to the sealer section 570. Adjusting means can be provided to vary the inclination, and therefore the lifting tendency, of the inclined axis rollers.

The next optional but preferred step is the fusion bonding of the open mesh sleeve material surrounding the pads for forming sleeve stock. As shown in Figures 30, 35 and 36, this step is preferably performed continuously employing heated, abutting, rotating metal belts while the pads are advanced longitudinally and with the sheath material surrounding them to form continuous running length sleeve stock surrounding the pads. When the pads and sheath material, thus configured, are received into sealer section 570, they slide through a longitudinally positioned tray 571 having sides 572 for confining the work material against lateral motion. The tray 571, which is supported upon transverse members 577, 578 held up by stanchions 573, 574 and connected by bases 575, 576 to subframe 533, also extends beneath the inclined axis rollers 562 and thus in certain circumstances may assist in holding the work in the desired orientation and at the appropriate elevation in alignment with the plane of abutment of the cooperating sealing belts.

The sealing belts are mounted in subframe 581, including first and second upright standards 582, 583 that support an overhead horizontal plate 584. Mounted in apertures in this plate are the various supports for the sealing belts which are positioned adjacent one another so as to squeeze the sheath material marginal edges 559 between them to cause said edges to fusion bond to one another and to draw the sheath material downstream by coordinated movement of the belts, i.e. at the same peripheral speed.

As shown in the drawings, there are right and left sealing belts 585, 586 having infeed idler units 587 in which there are vertical axles (not shown) on center lines 588 which support infeed belt rollers 589 at the appropriate elevation. The opposite ends of the sealing belts are supported by outfeed drive units 590 with vertical axles 591 supporting outfeed belt rollers 592. Resistance heaters 593 provided with suitable temperature control means (not shown) supply heat to heat transmission blocks 594 which frictionally engage the back sides of the moving sealing belts 585, 586 to maintain them at the proper temperature for sealing the sheath material. As shown in Figure 36, the sealing pressure of belts 585, 586 against the marginal edges 559 as well as the pressure of heat blocks 594 against the belts is maintained by a pair of pneumatic cylinders 595 underslung on plate 584 and having rams 596 and ram extensions 597 which can reciprocate perpendicular to the sealing axis of the belts and which support the blocks 594. The maximum inward projections of the blocks 594 and the above-mentioned pressures are governed by abutting contact between ram extensions 597 and a pair of adjustable stops 598 secured to fixed frame members 599.

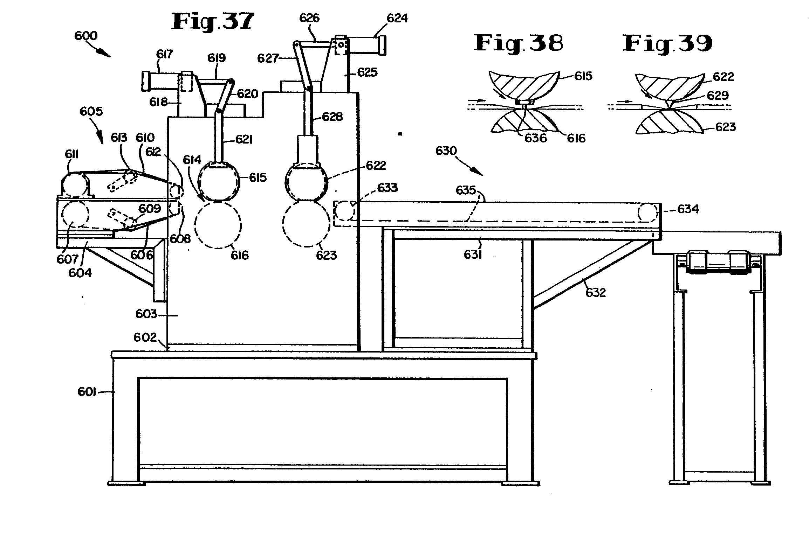

The resultant sleeve stock with pads within, received from sealer section 570, may be sealed transversely at one or both ends of the pads and cut to an appropriate length. For example, if the sleeve stock is sealed transversely, this can be done while the sleeve stock and pads are advancing longitudinally. Cutting may also be performed at longitudinal positions intermediate the pads with the pads and sleeve stock in longitudinal motion, thereby forming absorbent pads within sheaths having sheath extensions extending a predetermined distance beyond the ends of the pads, the sheath material being sealed at neither, one or both ends of the pads. The foregoing may be accomplished for example, by the sheath sealing and cutting section 600 of the preferred apparatus illustrated in Figures 37—39.

Section 600 includes a table 601 supporting a base 602 on which are mounted generally upright parallel plates 603 extending parallel to and on each side of the flow path of the continuous running length sleeve stock having spaced pads inside. An upstream frame extension 604 supporting a belt feeder 605 assists in moving the sleeve stock from sealer section 570 into this section of the apparatus. The belt feeder includes a lower belt 606 having its upper driving surface in contact with the bottom of the sleeve stock, the belt 606 being supported, driven and tensioned by lower infeed roller 607, lower outfeed roller 608 and tension roller 609. Upper infeed roller 611, upper outfeed roller 612 and upper tension roller 613 support, drive and tension the upper belt 610 with its lower driving surface in contact with the upper surface of the sleeve stock. Operation of belts 606 and 610 in unison feeds the sleeve stock at the same longitudinal rate of advancement at which it is discharged by the sealing belts 585 and 586 of sealer section 570. Belt feeder 605 delivers the sleeve stock to the nip 614 of sealing roller 615 with transverse sealing bar 636 and cooperating hardened polished plain cylinder 616 shown in greater detail in Figure 38. Sealing pressure can be applied and regulated with the aid of hydraulic cylinder 617 mounted in upper frame extension 618 and having its ram 619 pivotally connected to toggle 620 which vertically adjusts compression link 621, thereby exerting more or less sealing pressure on roller 615 through its shaft and bearings (not shown). There is a pair of the above-described combinations of hydraulic cylinder, frame extension, ram, toggle and compression link, one at each end of the shaft of bar seal roller 615, but only one such combination is visible in the drawings.

The cutting operation may be performed with a raised edge cutting roller 622 which is shown in Figure 37 and in greater detail in Figure 39, having its raised transverse cutting edges 629 spaced about the periphery of roller 622 at distance intervals equal to the desired sheath length. A cooperating lower cylinder 623 is provided, similar to cylinder 478 of Figure 30. Cutting pressure is applied and adjusted by hydraulic cylinder 624 on frame extension 625 with ram 626, toggle 627 and compression link 628 cooperating with corresponding components connected to the other end of the shaft (not shown) on which cutting roller 622 is mounted. The completed absorbent pads within sheaths having sheath extensions extending a predetermined distance beyond the ends of the pads are discharged from cutting roller 622 and lower cylinder 623 onto a discharge conveyor 630 having a belt 635 supported by infeed and outfeed rollers 633, 634 mounted in subframe 631 with frame extension 632, the latter being supported on the same table 601 which supports the sleeve sealing and cutting section 600.

Best Mode

At present it is considered best to form the brief as described in connection with Figures 1—4, above. For best results, it is considered advisable to form the pad from a composite of absorptive layers including layers of tissue and layers comprising water-soluble polymeric film, such as Dow water absorbent laminate material sold under the trade designations DWAL 30R, DWAL 30F, DWAL 35R and DWAL 35F, being generally characterized as approximately 50% by weight of polymer film sandwiched between two layers of paper tissue representing approximately the remaining 50% by weight of the laminate. The polymer is lightly cross-linked acrylic polymer as described in U.S. Patent 4,117,184. Said laminate has a polymeric film weight ranging from about 32 to about 38 grams per square meter, a laminate weight in the range of about 62 to about 69 grams per square meter and a liquid capacity (polymer film portion without tissue) in the range of about 35 to 48 grams of 1% salt solution per gram of polymer film. For the best tissue material we recommend a 2 ply tissue or wadding weighing 39 grams per square meter and sold by Kimberly Clark under the grade designation K 40.

For the sheath material it is presently preferred to employ Kendall Fiber Products Company Webline 146—039 NWF non-woven water permable fabric formed of polyester filaments which weighs 21 grams per square meter.

While the relative amounts of polymeric layer material and tissue employed in the pad are not particularly critical, they should preferably be sufficient to meet the overall absorption capacity specifications set forth under "Summary of the Invention". At present it appears best to employ the method of Figures 16—39 to produce a pad with the undulating configuration illustrated in Figure 29, using strips of raw material which are 31.8 centimeters wide to produce pads that are 25.4 centimeters long, the wrap and two-ply middle sheet being the aforementioned tissue and the two remaining sheets in the composite being the aforementioned Dow material.

One hundred fifteen 0.795 centimeter diameter substantially circular holes are punched in the composite in transverse rows with the centers of the holes in the respective rows being spaced apart approximately 2.5 centimeters both laterally and longitudinally, and with the number of holes in each row alternating between eleven and twelve. The holes in the eleven- and twelve-hole rows have their centers spaced, respectively, about 3.2 and 1.9 centimeters from the edges of the composite. These holes are of the hinge and flap configuration, corresponding in outline to the cutter of Figure 18A, with the paper hinges being about 2 mm wide and having their sides cut at a radius of about 1 mm. Most of the flaps are folded back by about 90° or more from the adjoining portion of the composite and do not therefore block the holes. In this embodiment the total area of the two sheets of polymeric material in the composite is 1,615 cm$^2$, the area per opening is 0.496 cm$^2$, the combined total number of openings in the two hydrophilic sheets is 230, the total open area is 114.2 cm$^2$, the ratio of the total open area to total hydrophilic sheet area (without deduction for the open area) is 0.071 and the ratio of the total number of openings in the hydrophilic sheet material to the total area of the hydrophilic sheet material is 0.142.

The design just described is presently considered to have acceptable production characteristics, absorption rate and capacity and to be best on grounds of esthetics and comfort which are of course major factors in the consumer acceptability of products of this type.

Use of Pad in Incontinence Care

To easily insert pads into the briefs shown in Figures 1—4, a sitting position is recommended. In so doing, the briefs can be stretched between the legs, just above the knees, and the moistureproof crotch panel 18 will then form a handy "work table". Insert the ends of the pad sheath extensions into the positioning loops 26, 28 to hold the pad in place. For extra security the sheath extensions may be threaded around and through the loops a second time. The briefs may now be pulled up to give a snug fit; and the waist band can be positioned for comfort. Because the thermoplastic layer 22 of panel 18 faces to the interior of pants 10, absorption of urine or other liquids by the body 12 of the pants is minimized which helps to prevent embarrassing odors and stains. When the pad needs changing, the briefs can be lowered to the knees, and the pad removed from the positioning loops in any convenient position, such as while sitting on a toilet. By firmly grasping one of the sheath extensions and shaking, the pad can be caused to fall through the open end of the sheath into the toilet bowl water. When the sheath is formed of material which does not disintegrate in water, which is usually the case, it is placed in a waste receptacle rather than the toilet. Depending on the absorbent material used, it may be preferable to wait about 30 or more seconds before flushing to allow for partial disintegration of the used pad. In the meantime, a new pad can be inserted and one's clothing repositioned. The illustrated embodiment has been specifically designed to meet the needs of the ambulatory person (walking and sitting). For complete protection at night one can use the pads with conventional diapers.

Advantages

The pads and briefs described herein may be embodied in a wide variety of forms without departing from the spirit of the invention. Depending on the exact form or embodiment selected, one or more, or in some cases all, of the following advantages may be attained. The fact that one or several of the following advantages may not be attained, does not however demonstrate that a particular embodiment is not within the scope of the invention.

It is an advantage of the preferred embodiments of the pad that because of their combined high absorption rate and capacity per unit of volume and area, they need only cover the target area and can thus be quite compact. This leads to subsidiary advantages, such as ease in carrying or concealment, for example in the user's purse, freedom from bulkiness when worn, and ease in changing and disposal, without sacrificing protection. A light dry weight compared to liquid weight capacity is also an advantage. When the pad includes the above described hydrophilic paper, its dry weight versus liquid capacity ratio is quite strikingly low. Moreover, the gel formed by a hydrophilic paper when wetted resists loss of liquid under small amounts of compression; this, combined with the moisture proof panel in the brief, reduces the opportunity for wetting and staining of clothing and gives the wearer more comfort (perception of dryness) and confidence. Preferred embodiments of the pad are non-linting, and offer reasonable cost versus liquid capacity. Moreover, the pads of the present embodiments do not make the tell-tale sound of rustling plastic associated with some other urinary incontinence care systems.

No special collection container is required for the pads, even when the hydrophilic paper is employed. The tissue paper readily disintegrates in water and the liquid gel and other components of the hydrophilic paper readily disperse on agitation and/or are biodegradable in conventional sewage treatment processes. The hydrophilic paper is not harmful to the skin and in certain embodiments has a tendency to fix the nitrogen compounds which give urine its characteristic odor and potential for irritation of the skin.

When the pad is used with a sheath, as above described, and the sheath is preferably of hydrophobic material, it tends to produce a feeling of dryness against the skin even though the pad within may contain substantial amounts of moisture. When the ends of the sheath extend beyond the ends of the pad, they provide a dry area to grasp when changing the pad and disposing of it, such as into a storage container or toilet.

No special collection container is required for the sheath either. Although hydrophobic polymers such as polypropylene may resist biodegradation, their hydrophobicity and the amine-fixing capabilities of the hydrophilic paper cause the sheath to remain relatively dry and free of odor-forming materials. Thus, the used sheath may be disposed of in a conventional domestic or institutional waste container.

The use of briefs offers certain advantages in its own right. First and foremost, they are effective, providing secure confinement of liquid by holding one or more pads securely in the proper position. The crotch portion of the brief supports the pad and assists in retarding or barring transmission of liquid and/or odors.

The briefs are safe. They are manufactured of readily available materials which are not harmful to the skin, nor are they harmed by skin oils, moisture or urine, and they are comfortable when worn.

Use of the briefs is convenient. As illustrated above, they can be put on and taken off with an up-down motion familiar to users of menstrual pad and belt combinations. However, the briefs can be made in forms which open in the front or side, with convenient and quick fastening arrangements such as "Velcro" (trademark) hook and loop fabric, to facilitate putting on and taking off, as well as pad changing. Thus in most cases this urinary incontinence care device can be used without help from another person.

The briefs are also esthetically acceptable. Because of their light weight and especially their lack of bulkiness they can be readily hidden beneath clothes. Like the pads, they can be made of materials which are noiseless during walking and other body motions. They are readily cleansed and remain durable during repeated washings in household or institutional clothes washers and dryers.

Other advantages of the pads and briefs will be apparent to persons of ordinary skill in the art, moreover, it can readily be appreciated that the use of the preferred pad and preferred brief separately or in combination can provide unique combinations of advantages not heretofore available in known urinary incontinence care products.

**Claims**

1. An absorbent pad (486) including at least one absorptive layer (132, 133) containing water-absorbent material including water-swellable material, having the capacity to absorb at least five times its own dry weight of clean water or other aqueous liquid characterised in that said at least one absorptive layer (132, 133) provides at least 50% of the total liquid absorption capacity of the pad, and has openings (134) which include hinge (283, 295) and flap (284) members at the perimeters of the openings (134), the flap members (284) extending outwardly from said at least one absorptive layer (132, 133).

2. An absorbent pad as claimed in Claim 1 characterised in that said at least one absorptive layer has the capacity to absorb at least 15 times its own dry weight of water or other aqueous liquid.

3. An absorbent pad as claimed in Claim 1 or Claim 2 characterised in that said at least one absorptive layer comprises a water-swellable polymeric material having the capacity to absorb at least 35 times its own dry weight of water or other aqueous liquid.

4. An absorbent pad as claimed in any preceding claim, characterised in that at least 60% of the total liquid absorption capacity of the pad is present in said at least one absorptive layer.

5. An absorbent pad as claimed in any preceding claim characterised in that said at least one absorptive layer comprises at least two layers (147, 148) of water absorbent polymeric material having between them a layer (150) of absorbent material of lesser absorption capacity per unit weight than said polymeric material.

6. An absorbent pad according to any preceding claim characterised in that said at least one absorptive layer as viewed in cross-section, has an undulating characteristic.

7. An absorbent pad according to any preceding claim characterised by an outer wrap of material (139) which has less absorption capacity, per unit weight, than that of the material of said at least one absorptive layer.

8. An absorbent pad according to any preceding claim characterised in that said openings (106, 134, 151B) have a sufficient average open area per opening and are distributed throughout a substantial portion of the available surface of said at least one absorptive layer (105, 132, 133, 147, 148) to provide an overall ratio of the total open area of said opening (106, 134, 151B) to the area of said portion (without deduction for the open area) in the range 0.002 to 0.25, the open area per opening being in the range of 0.02 to 1.3 cm², and the number of openings (106, 134, 151B) being in the range of 0.006 to 0.3 opening per cm² of the area of that portion of the material in which the openings (106, 134, 151B) are provided.

9. An absorbent pad according to Claim 8 characterised in that said ratio of total open area of the openings (106, 134, 151B) to the area of said portion is in the range of 0.002 to 0.20, the open area per opening is in the range of 0.05 to 1 cm², and the number of openings (106, 134, 151B) is in the range 0.006 to 0.25 openings per cm².

10. An absorbent pad according to any preceding claim characterised by including pieces which have been severed from said at least one absorptive layer in forming said openings (106, 134, 151B).

11. An absorbent pad according to any preceding claim characterised in that the pad is disposed within a sheath (169) which extends beyond at least one extremity of the pad (110, 130, 145, 486) to form one or more sheath extensions (175, 176), the sheath being formed of a material which substantially retains its structural integrity on exposure to human urine.

12. An absorbent pad according to any preceding claim characterised in that the pad is disposed within a sheath (169) which is free of permanent connections between itself and the pad and includes at least one open or openable end (174) through which the pad (110, 130, 145, 486) may be discharged from the sheath (169).

13. An absorbent pad according to any preceding claim characterised by having an overall absorptive capacity of at least 125 cc of natural or synthetic urine.

14. An absorbent pad according to Claim 12 characterised by having an overall, ultimate absorption capacity of at least 200 cc of natural or synthetic urine and having the property of absorbing at least 150 cc of such urine at an evacuation or application rate of substantially 5 cc per second.

15. Urinary incontinence care briefs characterised by including an absorbent pad according to any preceding claim, and comprising retaining means (26, 28) for removably retaining said pad (110, 130, 145, 486) in position for absorbing urine evacuated by the wearer.

16. A method of forming absorbent pads (486) comprising at least one absorptive layer (229, 249) of water absorbent material having the capability of absorbing at least five times its own dry weight of water or other aqueous liquid and having openings (134) therein, characterised in that the water absorbent material represents at least 50% of the total liquid absorption capacity of the raw materials processed into the pad and the method comprises forming said openings by cutting around a portion but not the entire periphery of a closed curve for forming hinge (283, 295) and flap (284) members, displacing said hinge (283, 295) and flap (284) members outwardly to form a significant open area (134), and cutting the resulting pad stock (308) transversely into sections to form pads (486).

17. A method according to Claim 16 characterised by forming a plurality of bends (329) in said at least one absorptive layer (229, 249), said bends (329) running lengthwise of and spaced at intervals across the width of said strip of water absorbent stock to form pad stocks (308) of greater thickness and narrower width than said at least one absorptive layer.

18. A method according to Claim 16 or Claim 17 characterised in that said openings (134) are formed throughout a substantial portion of the available surface of the absorptive layer (229, 249) for providing an overall ratio of the total open area of the opening (13) to the area of said portion (without deduction for the open area) in the range 0.002 to 0.25, the open area per opening (134) being in the range 0.02 to 1.3 cm², and the number of openings (134) being in the range of 0.006 to 0.3 openings per cm² of the area of that portion of the material in which the openings (134) are provided.

19. A method according to any of Claims 16 to 18 characterised in that the cutting step comprises cutting along lines which, as viewed in plan view, smoothly curve past the edges (285) of the hinge members (283, 295) to ends (286) which diverge from one another for inhibiting tear propagation in said at least one absorptive layer in and/or adjacent the hinge members (283, 295).

20. A method according to any of Claims 16 to 19 wherein said at least one absorptive layer material comprises a composite (308) having a plurality of layers (209, 229, 249), the method being characterised by forming coincident hinge (283, 295) and flap (284) members simultaneously in the plural layers (209, 229, 249) and displacing the plural flap (284) members so that they extend away from the layers (209, 229, 249) of which said hinge (283, 295) and flap (284) members are formed for locking the plural layers (209, 229, 249) together during subsequent longitudinal movement of the apertured composite (308) through the step of forming a pluraltiy of longitudinal bends (329) in said composite (308).

21. A method according to any of Claims 16 to 20 characterised by including the step of forming bends (329) in said at least one absorptive layer (229, 249) by drawing said layer in strip form (308) lengthwise through longitudinally oriented stationary members (325, 326) which converge both laterally and vertically, causing the strip (308) to contract laterally and alternately displacing adjacent portions of the contracting strip for forming an undulating cross-section therein.

22. A method according to any of Claims 16 to 21 characterised in that the strip of pad stock (308) is cut transversely into sections at longitudinal intervals to form pads (486) of predetermined length therefrom while said pad stock (308) is moving longitudinally in strip form, the method including the step of compressing a narrow band of material adjacent the trailing edge of each cut for causing collapsed and overlapping undulations and/or other layers to cling to one another with sufficient adherence to form a coherent leading edge (487) at the front of each cut pad section (486) which retains substantial structural integrity during subsequent advancement of the pad sections (486); advancing said pad sections (486) with coherent leading edges (487) longitudinally into contact with sheath material (499, 509) while the latter is advancing longitudinally at an accelerated rate relative to the rate of advancement of pad stock (308) during said transverse cutting of said pad stock (308), thereby causing the sections (486) of pad stock to travel longitudinally in contact with the sheath material (499, 509) with a predetermined space between said sections; and cutting the sheath material (499, 509) transversely between said pad sections (486) for forming pads (486) with sheaths (169) and sheath extensions (175, 176).

23. A method according to any of Claims 16 to 22 characterised in that the pad stock (308) is brought, in strip form, prior to or subsequent to cutting of said pad stock (308) transversely into sections of predetermined length, into contact with sheath material (499, 509) in strip form which is wider than said pad stock (308), the method including the steps of advancing the pad stock (308) and sheath material (499, 509) together longitudinally while bending the sheath material (499, 509) along a longitudinal line or lines for surrounding the pad stock (308) as viewed in transverse cross-section; bringing the advancing marginal edges (559) of the sheath material into contact with one another and so that said edges project outwardly relative to the nearest major surface of the pad stock (308); and securing said marginal edges (559) together to form said sheath material into sleeve stock surrounding said pad stock (308).

24. A method according to any of Claims 16 to 21 characterised in that the pad stock (308) is brought, in strip form, into contact with a strip of liquid permeable material (389, 399, 409) having less absorption capacity than said water absorbent material, the method including the steps of advancing the strips of pad stock (308) and liquid permeable material (389, 399, 409) together longitudinally while the liquid permeable material (389, 399, 409) is bent along one or more longitudinal lines for encircling the pad stock (308) as viewed in transverse cross-section, thereby forming a wrap of such permeable material (389, 399, 409) around the pad stock (308); simultaneously cutting said wrap (389, 399, 409) and pad stock (308) transversely at longitudinal intervals for forming pad sections (486); bringing the resultant wrapped pads (486) into contact with a strip of sheath material (499, 509) which is wider than said pads; moving the wrapped pads (486) longitudinally in contact with said sheath material (499, 509) while bending the latter along a longitudinal line or lines for encircling the pad sections (486) as viewed in transverse cross-section; and bonding the marginal edges (559) of the sheath material (499, 509) to one another for forming sleeve stock surrounding the wrapped pads (486).

**Patentansprüche**

1. Saugfähige Einlage mit zumindest einer saugfähigen Schicht (132, 133), die wasseraufsaugendes Material einschließlich durch Wasser aufquellbares Material enthält, das ein Saugvermögen von zumindest dem Fünffachen des eigenen Trockengewichtes für reines Wasser oder eine andere wäßrige Flüssigkeit aufweist, dadurch gekennzeichnet, daß die zumindest eine saugfähige Schicht (132, 133) wenigstens 50 % der gesamten Flüssigkeitssaugkapazität der Einlage zur Verfügung stellt und Öffnungen besitzt, denen Gelenkteile (283, 295) und Klappenteile (284) am Umfang der Öffnungen (134) zugeordnet sind, wobei sich die Klappenteile (284) der zumindest einen saugfähigen Schicht (132, 133) nach außen erstrecken.

2. Saugfähige Einlage nach Anspruch 1, dadurch gekennzeichnet, daß die zumindest eine saugfähige Schicht das Vermögen besitzt, wenigstens das Fünfzehnfache ihres Trockengewichtes an Wasser oder einer anderen wäßrigen Flüssigkeit aufzusaugen.

3. Saugfähige Einlage nach Anspruch 1 oder 2, dadurch gekennzeichnet, daß die zumindest eine saugfähige Schicht ein wasserquellbares Polymermaterial mit der Fähigkeit umfaßt, wenigstens das Fünfunddreißigfache seines eigenen Trockengewichtes an Wasser oder einer anderen wäßrigen Flüssigkeit aufzusaugen.

4. Saugfähige Einlage nach einem der vorhergehenden Ansprüche, dadurch gekennzeichnet, daß wenigstens 60 % der gesamten Flüssigkeitssaugkapazität der Einlage in der zumindest einen saugfähigen Schicht vorliegt.

5. Saugfähige Einlage nach einem der vorhergehenden Ansprüche, dadurch gekennzeichnet, daß die zumindest eine saugfähige Schicht wenigstens zwei Lagen (147, 148) von wasseraufsaugendem Polymermaterial umfaßt, zwischen denen eine Lage (150) von saugfähigem Material mit einer pro Gewichtseinheit geringeren Saugfähigkeit als das Polymermaterial angeordnet ist.

6. Saugfähige Einlage nach einem der vorhergehenden Ansprüche, dadurch gekennzeichnet, daß die zumindest eine saugfähige Schicht, im Querschnitt betrachtet, gewellt ist.

7. Saugfähige Einlage nach einem der vorhergehenden Ansprüche, gekennzeichnet, durch eine äußere Umhüllung aus einem Material (139), das eine geringere Saugfähigkeit pro Gewichtseinheit als das Material der zumindest einen saugfähigen Schicht aufweist.

8. Saugfähige Einlage nach einem der vorhergehenden Ansprüche, dadurch gekennzeichnet, daß die Öffnungen (106, 134, 151B) eine ausreichende mittlere Öffnungsfläche pro Öffnung besitzen und über einen wesentlichen Bereich der verfügbaren Oberfläche der zumindest einen saugfähigen Schicht (105, 132, 133, 147, 148) verteilt sind, um ein Gesamtverhältnis der Gesamtöffnungsfläche der Öffnungen (106, 134, 151B) zu der Fläche des Bereiches (ohne Abzug für die Öffnungsfläche) im Bereich von 0,002 bis 0,25 zur Verfügung zu stellen, wobei die offene Fläche pro Öffnung im Bereich von 0.002 bis 1,3 cm² liegt und die Anzahl der Öffnungen (106, 134, 151B) im Bereich von 0,006 bis 0,3 Öffnungen/cm² der Fläche des Bereiches des Materials, in dem die Öffnungen (106, 134, 151B) vorgesehen sind, liegt.

9. Saugfähige Einlage nach Anspruch 8, dadurch gekennzeichnet, daß das Verhältnis der gesamten Öffnungsfläche der Öffnungen (106, 134, 151B) zu der Fläche des Bereiches, in dem die Öffnungen vorgesehen sind, im Bereich von 0,002 bis 0,20 liegt, die Öffnungsfläche pro Öffnung im Bereich von 0,05 bis 1 cm² liegt und die Anzahl der Öffnungen (106, 134, 151B) im Bereich von 0,006 bis 0,25 Öffnungen/cm² liegt.

10. Saugfähige Einlage nach einem der vorhergehenden Ansprüche, gekennzeichnet durch den Einschluß von Stücken, die von der zumindest einen saugfähigen Schicht bei der Bildung der Öffnungen (106, 134, 151B) abgetrennt wurden.

11. Saugfähige Einlage nach einem der vorhergehenden Ansprüche, dadurch gekennzeichnet, daß die Einlage innerhalb einer Hülle (169) angeordnet ist, die sich über wenigstens einen Rand der Einlage (110, 130, 145, 486) hinaus erstreckt, um eine oder mehrere Hüllenverlängerungen (175, 176) zu bilden, wobei die Hülle aus einem Material gebildet wird, das im wesentlichen seine strukturelle Integrität behält, wenn es menschlichem Urin ausgesetzt wird.

12. Saugfähige Einlage nach einem der vorhergehenden Ansprüche, dadurch gekennzeichnet, daß die Einlage innerhalb einer Hülle (169) angeordnet ist, die frei von ständigen Verbindungen zwischen sich und der Einlage ist und daß die Hülle wenigstens ein offenes oder zu öffnendes Ende (174) aufweist, durch das die Einlage (110, 130, 145, 486) aus der Hülle (169) entfernt werden kann.

13. Saugfähige Einlage nach einem der vorhergehenden Ansprüche, gekennzeichnet durch eine Gesamtsaugfähigkeit von wenigstens 125 ml natürlichem oder synthetischem Urin.

14. Saugfähige Einlage nach Anspruch 12, gekennzeichnet durch eine gesamte, größte Saugkapazität von wenigstens 200 ml natürlichen oder synthetischen Urins und der Eigenschaft, wenigstens 150 ml dieses Urins bei einer Entleerungs- oder Anwendungsrate von im wesentlichen 5 ml/s aufzusaugen.

15. Medizinischer Blaseninkontinenzslip, dadurch gekennzeichnet, daß er eine saugfähige Einlage nach einem der vorhergehenden Ansprüche enthält und eine Halteeinrichtung (26, 28) zum entfernbaren Halten der Einlage (110, 130, 145, 486) in einer das Aufsaugen von durch den Träger abgegebenem Urin gewährleistenden Lage aufweist.

16. Verfahren zur Bildung von saugfähigen Einlagen (486) mit zumindest einer saugfähigen Schicht (229, 249) aus wasseraufsaugendem Material, das die Fähigkeit hat, wenigstens das Fünffache seines eigenen Trockengewichtes an Wasser oder einer anderen wäßrigen Flüssigkeit aufzusaugen und das Öffnungen (134) aufweist, dadurch gekennzeichnet, daß das wasseraufsaugende Material wenigstens 50 % des gesamten Flüssigkeitssaugvermögens des in der Einlage verarbeiteten Rohmaterials aufweist, mit den Verfahrensschritten Ausformen der Öffnungen durch Umfangsausschneiden eines Teiles, jedoch nicht entlang seiner geschlossenen Umfangskurve, so daß Gelenkteile (282, 295) und Klappenteile (284) gebildet werden, die Gelenkteile (283, 295) und Klappenteile (284) nach außen abgespreizt werden, um eine signifikante Öffnungsfläche (134) zu bilden und daß das erhaltene Einlagenmaterial (308) quer in einzelne Abschnitte geschnitten wird, um Einlagen zu bilden.

17. Verfahren nach Anspruch 16, gekennzeichnet durch die Ausbildung einer Mehrzahl von Falten (329) in der zumindest einen saugfähigen Schicht (229, 249), wobei die Falten (329) längs und im Abstand über die Breite des Streifens des wasseraufsaugenden Materials verlaufen, um Einlagenmaterialien (308) mit gegenüber der zumindest einen saugfähigen Schicht größerer Dicke und verringerter Breite zu schaffen.

18. Verfahren nach Anspruch 16 oder 17, dadurch gekennzeichnet, daß die Öffnungen (134) verteilt über einen wesentlichen Abschnitt der verfügbaren Oberfläche der saugfähigen Schicht (229, 249) gebildet werden, um ein Gesamtverhältnis der gesamten Öffnungsfläche der Öffnungen (13) zu der Grundfläche des Abschnittes (ohne Abzug für die Öffnungsfläche) im Bereich von 0,002 bis 0,25 zur Verfügung zu stellen, wobei die offene Fläche pro Öffnung (134) im Bereich von 0,02 bis 1,3 cm² liegt und die Zahl der Öffnungen (134) im Bereich von 0,006 bis 0,3 Öffnungen/cm² der Fläche des Abschnittes des Materials, in dem die Öffnungen (134) vorgesehen sind, liegt.

19. Verfahren nach einem der Ansprüche 16 bis 18, dadurch gekennzeichnet, daß der Verfahrensabschnitt des Ausschneidens das Schneiden entlang von Linien, die in Draufsicht betrachtet, gleichmäßig gekrümmt sind, umfaßt, vorbei an den Rändern (285) der Gelenkteile (283, 295) zu Enden (286), die sich voneinander weg erstrekken, um ein Weiterreißen in der zumindest einen saugfähigen Schicht in die Gelenkteile (238, 295) hinein und/oder ein Einreißen benachbart zu den Gelenkteilen (283, 295) zu verhindern.

20. Verfahren nach einem der Ansprüche 16 bis 19, worin das zumindest eine saugfähige Schichtmaterial einen Verbund (308) aus einer Mehrzahl von Lagen (209, 229, 249) umfaßt, gekennzeichnet durch die Ausformung übereinstimmender Gelenkteile (283, 295) und übereinstimmender Klappenteile (284) gleichzeitig in den mehreren Lagen (209, 229, 249) und Abspreizen der Klappenteile (284), so daß sie sich von den Lagen (209, 229, 249) weg erstrecken, aus denen die Gelenkteile (283, 295) und Klappenteile (284) herausgebildet werden, um die Lagen (209, 229, 249) während der nachfolgenden Längsbewegung des mit Öffnungen versehenen Verbundes (308) zur Bildung einer Mehrzahl von Längsfalten (329) in dem Verbund (308) miteinander in einen Verbindungseingriff zu bringen.

21. Verfahren nach einem der Ansprüche 16 bis 20, gekennzeichnet durch die Bildung von Falten (329) in der zumindest einen saugfähigen Schicht (229, 249) durch Hindurchziehen der Schicht in Streifenform (308) in Längsrichtung durch längsorientierte, stationäre Teile (325, 326), die sowohl seitlich als auch vertikal zusammenlaufen und veranlassen, daß sich der Streifen seitlich zusammenzieht und sich benachbarte Abschnitte des kontrahierenden Streifens zur Bildung eines gewellten Querschnittes wechselseitig gegeneinander verschieben.

22. Verfahren nach einem der Ansprüche 16 bis 21, dadurch gekennzeichnet, daß der Streifen aus Einlagenmaterial (308) in Längsintervallen quergeschnitten wird, um daraus Einlagen (486) mit vorbestimmter Länge zu bilden, während das Einlagenmaterial (308) sich in Längsrichtung in Streifenform bewegt, mit den Verfahrensschritten Zusammendrücken eines schmalen Materialstreifens an der Hinterkante jedes Schnittes, um flach zusammengedrückte, sich überlappende Wellungen zu bilden, und/oder zu veranlassen, daß andere Schichten mit ausreichender Haftung aneinander haften, um eine entsprechende, festgefügte, verschlossene Vorderkante (487) an der Vorderseite jedes geschnittenen Einlagenteiles (486) zu schaffen, die im wesentlichen ihre strukturelle Integrität während der darauffolgenden Vorwärtsbewegung der Einlagenabschnitte (486) behält, Vorwärtsbewegen der Einlagenteile (486) mit festgefügten, verbundenen Vorderkanten (487) in Längsrichtung zum Eingriff mit Hüllenmaterial (499, 509), wobei letzteres sich in Längsrichtung mit einer im Verhältnis zur Geschwindigkeit der Vorwärtsbewegung des Einlagenmaterials (308) größeren Geschwindigkeit während des Querschneidens des Einlagenmaterials (308) bewegt, wodurch bewirkt wird, daß sich die Abschnitte (486) des Einlagenmaterials in Längsrichtung in Berührungskontakt mit dem Hüllenmaterial (499, 509) mit einem vorbestimmten Abstand zwischen den Abschnitten bewegen, und Querschneiden des Hüllenmaterials (499, 509) zwischen den Materialabschnitten (486) zur Bildung von Einlagen (486) mit Umhüllungen (169) und Hüllenverlängerungen (175, 176).

23. Verfahren nach einem der Ansprüche 16 bis 22, dadurch gekennzeichnet, daß das Einlagenmaterial (308) in Streifenform vor oder nach dem Querschneiden des Einlagenmaterials (308) in Abschnitte von vorbestimmter Länge in Kontakt mit dem streifenförmigen Hüllenmaterial (499, 509) gebracht wird, das breiter ist als das Einlagenmaterial (308), mit den Verfahrensschritten gemeinsame Vorwärtsbewegung von Einlagenmaterial (308) und Hüllenmaterial (499, 509) in Längsrichtung, wobei das Hüllenmaterial (499, 509) entlang einer Längskante oder -kanten zum Einhüllen des Einlagenmaterials (308), im Querschnitt betrachtet, gebogen wird, Herstellen eines Berührungskontaktes zwischen den Seitenkanten (559) des Hüllenmaterials so, daß die Seitenkanten im Verhältnis zur benachbarten Hauptfläche des Einlagenmaterials (308) außen liegen, und Befestigung der Seitenkanten (559) verschließend aneinander, um das Hüllenmaterial als Materialhülse auszubilden, die das Einlagenmaterial (308) umgibt.

24. Verfahren nach einem der Ansprüche 16 bis 21, dadurch gekennzeichnet, daß das Einlagenmaterial (308) in Streifenform in Kontakt mit einem Streifen eines flüssigkeitsdurchlässigen Materials (389, 399, 409) gebracht ist, das eine geringere Saugfähigkeit als das wasseraufsaugende Material besitzt, mit den Verfahrensschritten gemeinsames Vorwärtsbewegen des Einlagenmaterialstreifens (308) und des Streifens aus flüssigkeitsdurchlässigem Material (389, 399, 409) in Längsrichtung, wobei das flüssigkeitsdurchlässige Material (389, 399, 409) entlang einer oder

24

mehrerer Längskanten zum Einhüllen des Einlagenmaterials (308), im Querschnitt betrachtet, gebogen wird, wodurch eine Umhüllung des durchlässigen Materials (389, 399, 409) um das Einlagenmaterial (308) gebildet wird, gleichzeitiges Querschneiden von Umhüllung (389, 399, 409) und Einlagenmaterial (308) in Längsintervallen zur Bildung von Einlagenteilen (486), Herstellen eines Berührungskontaktes der erhaltenen, umhüllten Einlagen (486) mit einem Streifen des Hüllenmaterials (499, 509), das breiter ist als die Einlage, Bewegen der umhüllten Einlagen (486) in Berührung mit dem Hüllenmaterial (499, 509) in Längsrichtung, wobei das letztere entlang einer Längskante oder -kanten zum Umfassen der Einlagenteile (486), im Querschnitt betrachtet, gebogen wird, und Verbinden der Seitenkanten (559) des Hüllenmaterials (499, 509) miteinander zur Bildung einer Materialhülse, die die umhüllten Einlagen (486) umgibt.

**Revendications**

1. Tampon absorbant (486) comprenant au moins une couche d'absorption (132, 133) contenant un matériau absorbant l'eau qui contient un matériau qui peut augmenter de volume sous l'action de l'eau, ayant la capacité d'absorber au moins cinq fois son propre poids à sec d'eau propre ou d'un autre liquide aqueux, caractérisé en ce que ladite couche d'absorption au moins (132, 133) constitue 50 % au moins de la capacité totale d'absorption de liquide du tampon, et a des ouvertures (134) qui comportent des volets (284) et des articulations (283, 295) au périmètre des ouvertures (134), les volets (284) étant dirigés vers l'extérieur par rapport à la couche d'absorption au moins (132, 133).

2. Tampon absorbant selon la revendication 1, caractérisé en ce que la couche d'absorption au moins a la propriété de pouvoir absorber au moins 15 fois son propre poids à sec d'eau ou d'un autre liquide aqueux.

3. Tampon absorbant selon l'une des revendications 1 et 2, caractérisé en ce que la couche d'absorption au moins contient un matériau polymère qui peut augmenter de volume sous l'action d'eau, ayant la propriété de pouvoir absorber au moins 35 fois son propre poids à sec d'eau ou d'un autre liquide aqueux.

4. Tampon absorbant selon l'une quelconque des revendications précédentes, caractérisé en ce que 60 % au moins de la capacité totale d'absorption de liquide du tampon est localisée dans au moins une couche d'absorption.

5. Tampon absorbant selon l'une quelconque des revendications précédentes, caractérisé en ce que la couche d'absorption au moins comprend au moins deux couches (147, 148) d'un matériau polymère absorbant l'eau, entre lesquelles est disposée une couche (150) d'un matériau absorbant ayant une moindre capacité d'absorption par unité massique que le matériau polymère.

6. Tampon absorbant selon l'une quelconque des revendications précédentes, caractérisé en ce que la couche d'absorption au moins a une configuration ondulée, en coupe.

7. Tampon absorbant selon l'une quelconque des revendications précédentes, caractérisé par une enveloppe externe (139) en un matériau qui a une capacité d'absorption par unité massique inférieure à celle du matériau de la couche d'absorption au moins.

8. Tampon absorbant selon l'une quelconque des revendications précédentes, caractérisé en ce que les ouvertures (106, 134, 151B) ont une section moyenne suffisante par ouverture et sont réparties sur une partie notable de la surface disponible de la couche d'absorption au moins (105, 132, 133, 147, 148) pour qu'elles donnent un rapport global de la section totale des ouvertures (106, 134, 151B) à la surface de cette partie (sans déduction de la surface ouverte) compris entre 0,002 et 0,25, la section par ouverture étant comprise entre 0,002 et 1,3 cm$^2$, et le nombre d'ouvertures (106, 134, 151B) étant compris entre 0,006 et 0,3 ouverture par cm$^2$ de surface de la partie du matériau dans laquelle les ouvertures (106, 134, 151B) sont ménagées.

9. Tampon absorbant selon la revendication 8, caractérisé en ce que le rapport de la surface totale des ouvertures (106, 134, 151B) à la surface de ladite partie est compris entre 0,002 et 0,20, la surface par ouverture est comprise entre 0,05 et 1 cm$^2$, et le nombre d'ouvertures (106, 134, 151B) est compris entre 0,006 et 0,25 ouverture par cm$^2$.

10. Tampon absorbant selon l'une quelconque des revendications précédentes, caractérisé en ce qu'il comprend des tronçons qui ont été séparés de la couche d'absorption au moins pour la formation des ouvertures (106, 134, 151B).

11. Tampon absorbant selon l'une quelconque des revendications précédentes, caractérisé en ce que le tampon est disposé dans une gaine (169) qui dépasse d'une extrémité au moins du tampon (110, 130, 145, 486) afin qu'elle forme un ou plusieurs prolongements (175, 176) de gaine, la gaine étant formée d'un matériau qui garde pratiquement son intégrité structurale lorsqu'il est exposé à de l'urine humaine.

12. Tampon absorbant selon l'une quelconque des revendications précédentes, caractérisé en ce que le tampon est placé dans une gaine (169) qui ne comporte pas de liaison permanente entre elle-même et le tampon et qui comprend au moins une extrémité (174) qui est ouverte ou peut l'être et par laquelle le tampon (110, 130, 145, 486) peut être évacué de la gaine (169).

13. Tampon absorbant selon l'une quelconque des revendications précédentes, caractérisé en ce qu'il a une capacité globale d'absorption d'au moins 125 cm$^3$ d'urine naturelle ou synthétique.

14. Tampon absorbant selon la revendication 12, caractérisé en ce qu'il possède une capacité globale finale d'absorption d'au moins 200 cm$^3$ d'urine naturelle ou synthétique et qu'il a la propriété d'absorber au moins 150 cm$^3$ d'une telle urine avec un débit d'évacuation ou d'application de 5 cm$^3$/s pratiquement.

15. Culotte hygiénique pour incontinence urinaire, caractérisée en ce qu'elle contient un tampon absorbant selon l'une quelconque des revendications précédentes, et comporte un dispositif de retenue (26, 28) destiné à retenir temporairement le tampon (110, 130, 145, 486) en position d'absorption de l'urine évacuée par la personne qui la porte.

16. Procédé de fabrication de tampon absorbant (486), comprenant au moins une couche d'absorption (229, 249) d'un matériau absorbant l'eau, apte à absorber au moins cinq fois son propre poids à sec d'eau ou d'un autre liquide aqueux et ayant des ouvertures (134), caractérisé en ce qu le matériau absorbant l'eau représente au moins 50 % de la capacité totale d'absorption de liquide des matières premières incorporées au tampon, et le procédé comprend la formation des ouvertures par découpe d'une partie de la périphérie d'une courbe fermée, mais non sa totalité, afin que des volets (284) et des articulations (283, 295) soient formés, le déplacement des volets (284) et des articulations (293, 295) vers l'extérieur afin qu'une surface ouverte importante (134) soit formée, et la découpe transversale du matériau résultant (308) du tampon en tronçons pour obtenir des patins (486).

17. Procédé selon la revendication 16, caractérisé par la formation de plusieurs plis (329) dans la couche absorbante au moins (229, 249), orientés longitudinalement dans celle-ci et étant transversalement espacés à certains intervalles de manière à former des matériaux (308) de patins d'épaisseur supérieur et de largeur inférieure à celle de la couche d'absorption au moins.

18. Procédé selon l'une des revendications 16 et 17, caractérisé en ce que les ouvertures (134) sont formées sur une partie notable de la surface disponible de la couche d'absorption (229, 249) afin qu'elles donnent un rapport global de la surface totale des ouvertures (13) à la surface de ladite partie (sans déduction de la surface ouverte) comprise entre 0,002 et 0,25, la surface ouverte par ouverture (134) étant comprise entre 0,02 et 1,3 cm² et la nombre d'ouvertures (134) étant compris entre 0,006 et 0,3 ouverture par cm² de la surface de la partie du matériau dans laquelle les ouvertures (134) sont ménagées.

19. Procédé selon l'une quelconque des revendications 16 à 18, caractérisé en ce que l'étape de découpe comprend une découpe suivant des lignes qui, en plan, sont courbées progressivement au niveau des bords (285) des articulations (283, 295) vers des extrémités (286) qui divergent l'une de l'autre afin que la propagation des déchirures dans la couche d'absorption au moins soit empêchée dans les articulations et/ou près de celles-ci (283, 295).

20. Procédé selon l'une quelconque des revendications 16 à 19, dans lequel le matériau de la couche d'absorption au moins comprend un matériau composite (308) ayant plusieurs couches (209, 229, 249), le procédé étant caractérisé par la formation simultanée de volets (284) et d'articulations (283, 295) qui coïncident, simultanément dans plusieurs couches (209, 229, 249) et le déplacement des volets (284) afin qu'ils s'écartent desdites couches (209, 229, 249) dont les volets (284) et les articulations (283, 295) sont formés afin qu'ils bloquent les couches (209 229, 249) les unes par rapport aux autres lors d'un déplacement longitudinal ultérieur du matériau composite (308) ayant les ouvertures dans l'étape de formation des plis longitudinaux (329) dans le matériau composite (308).

21. Procédé selon l'une quelconque des revendications 16 à 20, caractérisé en ce qu'il comprend l'étape de formation de plis (329) dans la couche d'absorption au moins (229, 249) par étirage longitudinal de la couche sous forme d'une bande (308) entre des organes fixes disposés longitudinalement (325, 326) qui convergent à la fois latéralement et verticalement, si bien que la bande (308) se contracte latéralement, et le déplacement alterné de parties voisines de la bande qui se contracte afin d'obtenir une section ondulée.

22. Procédé selon l'une quelconque des revendications 16 à 21, caractérisé en ce que la bande du matériau (308) pour tampon est découpée transversalement en tronçons à certains intervalles longitudinaux afin qu'elle forme des tampons (486) de longueur prédéterminée, pendant que le matériau (308) pour tampon se déplace longitudinalement sous forme d'une bande, le procédé comprenant l'étape de compression d'une bande étroite de matériau près du bord postérieur de chaque découpe afin que les ondulations et/ou d'autres couches affaissées et en recouvrement d'accrochent les unes aux autres avec une adhérence suffisante pour qu'elles forment un bord antérieur cohérent (487) à l'avant de chaque tronçon découpé (486) de tampon gardant une bonne intégrité lors de l'avance ultérieure des tronçons de tampon (486), l'avance des tronçons de tampons (486) avec les bords antérieurs cohérents (487) longitudinalement au contact d'un matériau de gaine (499, 509) pendant que ce dernier avance longitudinalement avec une vitesse supérieure à la vitesse d'avance du matériau pour tampon (308) pendant la découpe transversale du matériau pour tampon (308) si bien que les tronçons (486) du matériau pour tampon se déplacent longitudinalement au contact du matériau de gaine (499, 509) avec un espace prédéterminé entre les tronçons, et la découpe du matériau de gaine (499, 509) transversalement entre les tronçons de tampon (486) afin de former des tampons (486) ayant des gaines (169) et des prolongements de gaine (175, 176).

23. Procédé selon l'une quelconque des revendications 16 à 22, caractérisé en ce que le matériau pour tampon (308), sous forme d'une bande et avant ou après découpe du matériau pour tampon (308) transversalement en tronçons de longueur prédéterminée, est mis au contact d'un matériau de gaine (499, 509) sous forme d'une bande ayant une largeur supérieure à celle du matériau pour tampon (308), le procédé comprenant les étapes d'avance du matériau pour tampon (308) et du matériau de gaine (499, 509) ensemble en direction longitudinale avec pliage du matériau de gaine (499, 509) suivant une ou plusieurs lignes longitudinales afin que le matériau pour tampon (308) soit entouré en coupe transversale, de mise des bords marginaux (559) du matériau de gains qui avance au contact l'un de l'autre et de manière que les bords dépassent vers l'extérieur par rapport à la plus proche des grandes faces du matériau pour tampon (308), et de fixation mutuelle des bords marginaux (559) afin que le matériau de gaine forme un matériau sous forme d'un manchon entourant le matériau de tampon (308).

24. procédé selon l'une quelconque des revendications 16 à 21, caractérisé en ce que le matériau pour tampon (308), sous forme d'une bande, est mis au contact d'une bande d'un matériau perméable aux liquides (389, 399, 409) ayant une capacité d'absorption inférieure à celle du matériau absorbant l'eau, le procédé comprenant les étapes d'avance des bandes du matériau de tampon (308) et du matériau perméable aux liquides (389, 399, 409) en direction longitudinale et ensemble, alors que le matériau perméable aux liquides (389, 399, 409) est plié le long d'une ou plusieurs lignes longitudinales afin qu'il entoure le matériau pour tampon (308), en coupe transversale, avec formation d'une enveloppe du matériau perméable (389, 399, 409) autour du matériau pour tampon (308), de découpe simultanée de l'enveloppe (389, 399, 409) et du matériau pour tampon (308) en direction transversale, à certains intervalles longitudinaux afin de former des tronçons de tampon (486) de mise des tampons enveloppés résultants (486) au contact d'une bande d'un matériau de gaine (499, 509) qui a une largeur supérieure à celle des tampons, de déplacement des tampons enveloppés (486) en direction longitudinale au contact du matériau de gaine (499, 509) avec pliage de ce dernier suivant une ou plusieurs lignes longitudinales afin d'entourer les tronçons de tampon (486), en coupe transversale, et de fixation mutuelle des bords marginaux (559) du matériau de gaine (499, 509) afin qu'ils forment un matériau sous forme d'un manchon entourant les tampons enveloppés (486).

Fig.1

Fig.3

Fig.2

Fig.4

1

# Fig.5

106

105

106

# Fig.7

113

114

116

114

114

110

115

# Fig.8

# Fig.6

110

107

105A
105B

108

110

113

113

## Fig. 9

## Fig. 12

## Fig. 10

## Fig. 11

**Fig.13**

**Fig.14**

**Fig.15**

0 062 495

Fig.16

Fig.18

Fig.19

Fig.17

Fig.20

Fig.18A

0 062 495

Fig.21

## Fig.22

## Fig.23

## Fig.24

Fig. 25

Fig. 26

## Fig.27

## Fig.28

## Fig.29

## Fig.25A

## Fig.31

## Fig.31A

Fig.30

Fig.33

Fig.34

Fig.32

# Fig. 35

570
588
587
584
589
585
566
565
562
559
559
567
564
566
563
572
577
571
573
575

# Fig. 36

581
584
598
598
595
595
599
599
586
585
596
597
594
594
597
596
593
593
571
572
559
572
577

Fig.37  Fig.38  Fig.39